# EUROPEAN PATENT APPLICATION

(11) **EP 4 223 350 A1**
(43) Date of publication of application: **09.08.2023**
(21) Application number: 21875896.9
(22) Date of filing: 01.10.2021
(51) Int. Cl.: A61M 25/00

(54) **LONG MEDICAL INSTRUMENT AND PRODUCTION METHOD THEREFOR**

(30) Priority: 02.10.2020 WO PCT/JP2020/037644
(71) Applicant: Asahi Intecc Co., Ltd., Seto-shi, Aichi 489-0071 (JP)
(72) Inventor: NOGUCHI, Naoki, Seto-shi, Aichi 489-0071 (JP); KOSUGI, Tomoki, Seto-shi, Aichi 489-0071 (JP); NAKANISHI, Yuta, Seto-shi, Aichi 489-0071 (JP); GOTO, Kenta, Seto-shi, Aichi 489-0071 (JP); TAKAHASHI, Hiroaki, Seto-shi, Aichi 489-0071 (JP); KAKUMU, Naoki, Seto-shi, Aichi 489-0071 (JP); MURAKAMI, Tomoaki, Seto-shi, Aichi 489-0071 (JP)
(74) Representative: Winter, Brandl - Partnerschaft mbB
(86) International application number: PCT/JP2021/036524
(87) International publication number: WO 2022/071600

(57) **Abstract**

An elongated medical instrument includes: a base material part; and an outer layer part made of a resin based on a resin suspension that is coated on an outer peripheral surface of the base material part; where a surface of the outer layer part has irregularly disposed recesses formed from cracks generated by drying of the resin suspension, and protrusions formed with an outer peripheral surface formed on an outer peripheral side of the recesses.

## Description

### CROSS REFERENCE TO RELATED APPLICATIONS

This application is a continuation application of International Application No. PCT/JP2021/036524, filed October 01, 2021, which claims priority to International Application No. PCT/JP2020/037644, filed October 02, 2020. The contents of these applications are incorporated herein by reference in their entirety.

### TECHNICAL FIELD

The present disclosure relates to an elongated medical instrument.

### BACKGROUND

In order to achieve low-friction properties when inserting an elongated medical instrument such as a guide wire or a catheter into the body, there is known a technique in which the surface of an elongated medical instrument is coated with a hydrophilic resin or a fluororesin, and recesses and protrusions are formed on the surface (Patent Literatures 1 and 2). Such a technique is capable of reducing the contact area between an elongated medical instrument and a tubular tissue inside the body. When inserting an elongated medical instrument into a blood vessel, this technique is effective because the blood vessel itself in healthy, non-lesioned areas can maintain a tubular structure due to the blood pressure, and because blood also has a relatively low viscosity.

### CITATION LIST

### Patent Literature

Patent Literature 1: International Publication No. 2009/081844
Patent Literature 2: Japanese Unexamined Patent Application Publication No. 2008-125523

### SUMMARY

### TECHNICAL PROBLEM

An elongated medical instrument capable of improving the low-friction properties when passing through living tubular tissue having high friction is investigated. The friction when an elongated medical instrument is inserted into the body can sometimes be high. For example, among the living tubular tissue present in the body, the friction when an elongated medical instrument is inserted into the body increases in strongly curved blood vessel sites such as the aortic arch or internal carotid artery, in the gastrointestinal tract, which has a higher flexibility than a normal blood vessel, and in lesioned blood vessel sites and the gastrointestinal tract, in which liquids that are more viscous than blood are present.

Therefore, the present disclosure provides an elongated medical instrument capable of improving the low-friction properties when passing through living tubular tissue, and a production method therefor.

### SOLUTION TO PROBLEM

An elongated medical instrument according to an aspect of the present disclosure includes: a base material part; and an outer layer part made of a resin based on a resin suspension that is coated on an outer peripheral surface of the base material part; wherein a surface of the outer layer part has irregularly disposed recesses formed from cracks generated by drying of the resin suspension, and protrusions formed with an outer peripheral surface formed on an outer peripheral side of the recesses.

In the present disclosure, the term "irregularly disposed recesses" refers to recesses that are not regulated by a predetermined pattern as a whole, rather than recesses that are formed based on a combination of one or more predetermined patterns. That is, the recesses formed in the resin-made outer layer part of the elongated medical instrument have predetermined variations in depth, opening width, and spacing formed between the plurality of recesses that are greater than the variations that occur during production.

An elongated medical instrument according to another aspect of the present disclosure includes: a base material part; and an outer layer part made of a resin and provided on an outer peripheral surface of the base material part; wherein a surface of the outer layer part has irregularly disposed recesses, and protrusions formed with an outer peripheral surface on an outer peripheral side of the recesses, the outer layer part is formed by drying a resin suspension after being coated on a surface of the base material part, and the recesses are formed by drying a suspension coated on the outer layer part.

The recesses may be linearly and irregularly disposed in the circumferential direction and in the longitudinal axis direction.

The resin suspension forming the outer layer part may contain fluororesin particles.

The fluororesin particles may be perfluoroalkoxyalkane particles.

The elongated medical instrument may also be a guide wire including the base material part, a first region, in which a coil member is disposed on an outer periphery in a circumferential direction of the base material part, and a second region, in which an outer layer part made of a resin is disposed on an outer periphery in a circumferential direction of the base material part, which is proximal to the first region, wherein the recesses are provided in the second region.

The base material part may include a tubular body, and a reinforcing body formed on an outer peripheral side of the tubular body, and the outer layer part may cover the tubular body and the reinforcing body.

An adhesive layer may be provided on an outer peripheral side of the reinforcing body and between the outer layer part, and formed of a resin having increased adhesion with the reinforcing body.

The resin having increased adhesion may be a fluororesin having an adhesive functional group.

The fluororesin having an adhesive functional group may be a perfluoroalkoxyalkane having an adhesive functional group.

The base material part may include a tubular body, a reinforcing body formed on an outer peripheral side of the tubular body, and an intermediate resin layer formed so as to cover the tubular body and bury the reinforcing body, and the outer layer part may cover the tubular body and the reinforcing body.

The intermediate resin layer and the outer layer part may be formed of different resins, and the adhesive layer formed of a resin having increased adhesion with the intermediate resin layer may be formed between the intermediate resin layer and the outer layer part.

The resin forming the adhesive layer may be a fluororesin having an adhesive functional group.

The fluororesin having an adhesive functional group may be a perfluoroalkoxyalkane having an adhesive functional group.

A production method of an elongated medical instrument according to yet another aspect of the present disclosure includes: a first step of preparing a base material part; a second step of coating a resin suspension on an outer periphery of the base material part; a third step of drying the resin suspension coated on the base material part; and a fourth step of sintering a coating of the resin suspension; wherein in the third step, cracks are formed in a coating of the dried resin suspension, and after the fourth step, recesses are formed in a surface due to the cracks.

The resin suspension may contain fluororesin particles.

The fluororesin particles may be perfluoroalkoxyalkane particles.

A fifth step of forming an adhesive layer made of a resin having increased adhesion to the base material part may be provided between the first step and the second step.

The resin having increased adhesion to the base material part may be a fluororesin layer having an adhesive functional group.

The fluororesin having an adhesive functional group may be a perfluoroalkoxyalkane having an adhesive functional group.

### EFFECT OF THE DISCLOSED EMBODIMENTS

According to the present disclosure, the contact area with a tubular tissue inside the body can be reduced.

### BRIEF DESCRIPTION OF DRAWINGS

FIG. 1 is a schematic diagram of a guide wire according to an embodiment of the present disclosure, and a catheter using the guide wire.
FIG. 2 is a longitudinal sectional view showing a portion of a guide wire as an example of an elongated medical instrument.
FIG. 3 is an explanatory diagram showing the process of forming recesses on the surface of a guide wire.
FIG. 4 is an external view showing a state where cracks have formed on the surface of a guide wire.
FIG. 5 is an external view showing enlarged a portion of a guide wire.
FIG. 6 is an external view showing enlarged a portion of a guide wire according to a comparative example.
FIG. 7 is a perspective view showing a device that measures the outer diameter of a guide wire.
FIG. 8 is a graph of data obtained by measuring the outer diameter dimension of a guide wire having recesses on the outer layer part along the axial direction.
FIG. 9 is a waveform diagram obtained by an FFT analysis of the measurement data in FIG. 8.
FIG. 10 is a table showing FFT analysis results of the measurement data in FIG. 8.
FIG. 11 is a table showing the results of measuring the outer diameter dimension of a guide wire having recesses on the outer layer part.
FIG. 12 is a graph of data obtained by measuring the outer diameter dimension of a guide wire according to a comparative example along the axial direction.
FIG. 13 is a waveform diagram obtained by an FFT analysis of the measurement data in FIG. 12.
FIG. 14 is a table showing FFT analysis results of the measurement data in FIG. 12.
FIG. 15 is a table of the results of measuring the outer diameter dimension of a guide wire according to a comparative example.
FIG. 16 is a table showing the results of measuring the outer diameter dimension of the tapered portion of a guide wire.
FIG. 17 is an analysis result table for another case where irregular recesses are formed on the surface of a guide wire.
FIG. 18 is a graph showing an external dimension measurement.
FIG. 19 is a graph showing FFT analysis results of the external dimension measurement data.
FIG. 20 is an analysis result table for yet another case where irregular recesses are formed on the surface of a guide wire.
FIG. 21 is a graph showing an outer diameter dimension measurement.
FIG. 22 is a graph showing FFT analysis results of the outer diameter dimension measurement data.
FIG. 23 is a graph showing a comparison of the sliding resistance between a guide wire having recesses on the surface and a guide wire having no recesses on the surface.
FIG. 24 is a longitudinal sectional view of a medical tube according to Example 2.
FIG. 25 is an explanatory diagram showing the process of forming recesses on the surface of a medical tube.
FIG. 26 is a longitudinal sectional view of a medical tube according to Example 3.
FIG. 27 is an explanatory diagram showing the process of forming recesses on the surface of a medical tube.
FIG. 28 is a table obtained by an FFT analysis of measurement data for the far side of a guide wire according to Example 4.
FIG. 29 is a table obtained by an FFT analysis of measurement data for the near side of a guide wire.
FIG. 30 is a table showing measurement data of a guide wire.
FIG. 31 is a table showing data measuring the variation of the outer layer part (labeled as outer layer resin) excluding the core wire from the guide wire.
FIG. 32 is a table showing measurement data for another form of a guide wire.
FIG. 33 is a table showing data measuring the variation of the outer layer part (labeled as outer layer resin) excluding the core wire from the guide wire.
FIG. 34 is a table showing measurement data for yet another form of a guide wire.
FIG. 35 is a table showing data measuring the variation of the outer layer part (labeled as outer layer resin) excluding the core wire from the guide wire.
FIG. 36 is measurement data and an enlarged external view of the far side of the guide wire corresponding to FIG. 30.
FIG. 37 is measurement data and an enlarged external view of the near side of the guide wire corresponding to FIG. 30.
FIG. 38 is measurement data and an enlarged external view of the guide wire corresponding to FIG. 32.
FIG. 39 is measurement data and an enlarged external view of the guide wire in FIG. 34.
FIG. 40 is a table showing MPF and MDF values analyzed for various guide wires.
FIG. 41 is an explanatory diagram showing the table in FIG. 40 as a graph.
FIG. 42 is a table obtained by analyzing measurement data for a guide wire according to Example 5.
FIG. 43 is an explanatory diagram showing an enlarged external view and outer diameter data of a guide wire having a diameter of 0.79 mm.
FIG. 44 is an explanatory diagram showing an enlarged external view and outer diameter data of another guide wire having a diameter of 0.79 mm.
FIG. 45 is an explanatory diagram showing an enlarged external view and outer diameter data of a guide wire having a diameter of 0.70 mm.
FIG. 46 is an explanatory diagram showing an enlarged external view and outer diameter data of a guide wire having a diameter of 0.55 mm.
FIG. 47 is an explanatory diagram showing an enlarged external view and outer diameter data of a guide wire having a diameter of 0.415 mm.
FIG. 48 is an explanatory diagram showing an enlarged external view and outer diameter data of another guide wire having a diameter of 0.415 mm.
FIG. 49 is an explanatory diagram showing an enlarged external view and outer diameter data of yet another guide wire having a diameter of 0.415 mm.
FIG. 50 is an explanatory diagram showing an enlarged external view and outer diameter data of another guide wire having a diameter of 0.415 mm.
FIG. 51 is an explanatory diagram showing an enlarged external view and outer diameter data of still yet another guide wire having a diameter of 0.415 mm.
FIG. 52 is an explanatory diagram showing an example of a blood vessel in which a guide wire is used.
FIG. 53 is an explanatory diagram showing a state in which a guide wire is used in a lower extremity blood vessel model.
FIG. 54 is a graph showing the MPF for cases where the resin coating speed with respect to a 0.415 mm guide wire is varied.
FIG. 55 is a graph showing, for each resin coating speed of a 0.415 mm guide wire, a comparison between the sliding resistance value when used in a location where the simulated blood vessel is strongly curved, and the sliding resistance value when used in a location where the simulated blood vessel is gently curved.
FIG. 56 is a table showing the MPF of a plurality of guide wires prepared with varying resin coating speeds, and the sliding resistance values of the plurality of guide wires when used in a strongly curved portion of a simulated blood vessel.
FIG. 57 is a table showing the MPF of a plurality of guide wires prepared with varying resin coating speeds, and the sliding resistance values of the plurality of guide wires when used in a gently curved portion of a simulated blood vessel.
FIG. 58 is a graph comparing the measurement data in FIG. 56 and the measurement data in FIG. 57 according to the type of curvature of the simulated blood vessel.
FIG. 59 is a graph showing a comparison between the sliding resistance value when a guide wire is coated with PFA and the sliding resistance value when coated with PTFE.
FIG. 60 is a table showing the depth dimension of the recesses (wrinkle depth), MPF, MDF, film thickness, and cumulative power value for the catheter according to Example 6.
FIG. 61 is an enlarged view of the exterior of the near side of a catheter (labeled as tube_upper).
FIG. 62 is a table showing MDF and MPF values.
FIG. 63 is a graph showing the outer diameter of a catheter along the length direction of the catheter.
FIG. 64 is a graph showing a portion of FIG. 63 enlarged.
FIG. 65 is a table showing analysis results of data for the near side of a catheter.
FIG. 66 is a table showing measurement results of outer diameter data for the near side of a catheter.
FIG. 67 is an enlarged view of the exterior near the middle of a catheter (labeled as tube_middle).
FIG. 68 is a table showing MDF and MPF values.
FIG. 69 is a graph showing the outer diameter of a catheter along the length direction of the catheter.
FIG. 70 is a graph showing a portion of FIG. 69 enlarged.
FIG. 71 is a table showing analysis results of data near the middle of a catheter.
FIG. 72 is a table showing measurement results of outer diameter data near the middle of a catheter.
FIG. 73 is an enlarged view of the exterior of the far side of a catheter (labeled as tube_lower).
FIG. 74 is a table showing MDF and MPF values.
FIG. 75 is a graph showing the outer diameter of a catheter along the length direction of the catheter.
FIG. 76 is a graph showing a portion of FIG. 75 enlarged.
FIG. 77 is a table showing analysis results of data for the far side of a catheter.
FIG. 78 is a table showing measurement results of outer diameter data for the far side of a catheter.

### DETAILED DESCRIPTION OF EMBODIMENTS

In one aspect of the present disclosure, as described below, a plurality of recesses are provided on the surface of the elongated medical instrument in order to reduce the contact area with a tubular tissue in the body. The plurality of recesses are linearly formed in the circumferential direction and the longitudinal axis direction. As a result, the elongated medical instrument according to the present disclosure has a lower frictional resistance when passing through living tubular tissue having high friction. Because the elongated medical instrument according to the present disclosure has a plurality of recesses on the surface, the contact area between the surface of the elongated medical instrument and the inner wall of a blood vessel can be reduced. That is, low-friction properties can be achieved when the elongated medical instrument according to the present disclosure is inserted, for example, into a strongly curved blood vessel site.

In the present embodiment, an outer layer part is formed by coating a resin suspension on an outer peripheral surface of a base material part, and the outer layer part has irregularly disposed recesses formed by drying the resin suspension, and protrusions formed with an outer peripheral surface on an outer peripheral side of the recesses.

As one aspect, an elongated medical instrument includes: a base material part; and an outer layer part made of a resin and provided on an outer peripheral surface of the base material part; wherein a surface of the outer layer part has irregularly disposed recesses, and protrusions formed with an outer peripheral surface on an outer peripheral side of the recesses, the outer layer part is formed by drying a resin suspension after being coated on a surface of the base material part, and the recesses are formed by drying a suspension coated on the outer layer part.

The recesses may be linearly formed, and irregularly disposed in the circumferential direction and the longitudinal axis direction of the elongated medical instrument. The resin suspension forming the outer layer part may contain fluororesin particles.

The elongated medical instrument of the present disclosure is capable of reducing the frictional resistance when inserted into a predetermined tubular tissue. The predetermined tubular tissue, for example, is the gastrointestinal tract, which has a higher flexibility than a normal blood vessel, or a lesioned blood vessel site or the gastrointestinal tract, in which liquids that are more viscous than blood are present.

In another viewpoint of the present disclosure, an elongated medical instrument having improved sliding properties is provided. In this viewpoint, the irregularly disposed recesses on the surface of the outer layer part are capable of reducing the sliding resistance with a biological wall, such as the inner wall of a blood vessel, that makes contact with the elongated medical instrument, or the sliding resistance with the inner wall of a lumen of another elongated medical instrument through which the elongated medical instrument is inserted.

Hereinafter, an embodiment of the present disclosure will be described. The elongated medical instrument according to the present embodiment is configured as an elongated medical instrument such as a guide wire 1 or 1A, or as a catheter 2. The elongated medical instrument includes a base material part 11 or 11A, and an outer layer part 12 or 12A made of a resin and provided on the surface of the base material part 11 or 11A. The recesses 13 are, as described below, irregularly formed in the circumferential direction and the longitudinal axis direction of the elongated medical instrument when the outer layer part 12 or 12A is formed by coating a resin suspension on the base material part 11 or 11A. The recesses 13 are irregularly formed based on cracks 130 that form when drying the resin suspension coated on the base material part 11 or 11A. In one viewpoint, the surface of the outer layer part 12 or 12A is provided with irregularly disposed recesses, and protrusions 14 formed with an outer peripheral surface formed on an outer peripheral side of the recesses.

In another viewpoint, the elongated medical instrument is a guide wire 1 including a base material part 11 or 11A, a first region A1, in which a coil member 23 is disposed on an outer periphery in a circumferential direction of the base material part 11 or 11A, and a second region A2, in which an outer layer part 12 or 12Amade of a resin is disposed on an outer periphery in a circumferential direction of the base material part 11 or 11A, which is proximal to first region A1 (the hand side or near side of the operator). The guide wire 1 has recesses in the second region A2.

The base material part 11B may have a tubular body 111B, and a reinforcing body 113B formed on an outer peripheral side of the tubular body 111B, and the outer layer part 12B may cover the tubular body 111B and the reinforcing body 113B.

In yet another viewpoint, the recesses 13 are irregularly disposed on a substantially flat surface 121 of the outer layer part 12 or 12A. The substantially flat surface 121 is a surface that is flat and free of recesses and protrusions, with the exception of the recesses 13 according to the present disclosure. In other words, the substantially flat surface 121 is a surface that would have no recess or protrusion if the recesses 13 according to the present disclosure were absent there on. When an elongated medical instrument such as the guide wire 1 or the catheter 2 includes an uneven portion having a coil shape or the like, recesses and protrusions appear on a surface region corresponding to the uneven portion.

That is, the substantially flat surface 121 may be a surface region corresponding to the region of the outer layer part 12 or 12A under which under which the coil is not disposed. For example, in a case where the elongated medical instrument 1 is a balloon catheter, the region which is proximal to the balloon (the region in which the balloon is not formed) is an example of the substantially flat surface 121. In a case where the elongated medical instrument 1 is a guide wire, the region which is proximal to the area under which the coil on the distal end of the guide wire is formed (the region under which the coil is not provided) is an example of the substantially flat surface 121.

Those cases where the recesses 13 according to the present disclosure are irregularly formed on a surface other than the substantially flat surface 121 are also included in the scope of the present disclosure. For example, an elongated medical instrument in which the surface of the outer layer part 12 that corresponds to the coil shape is formed with recesses 13 in addition to the recesses and protrusions due to the coil shape is a type of elongated medical instrument according to the present disclosure.

The shape of the recesses 13 may be that of an irregularly shaped linear portion. The shape of the recesses 13 may include a spatial frequency of 3 to 10 (l/mm).

The irregularly disposed (formed) and irregularly shaped recesses 13 can be expressed as irregularly shaped linear portions 13 that are irregularly formed on the surface of the outer layer part 12 or 12A. Alternatively, the irregularly shaped recesses 13 can be described as irregularly shaped linear portions 13 originating from the cracks 130 generated in the outer layer part 12 or 12A.

Therefore, the elongated medical instrument according to the present embodiment may be expressed as including, for example, the base material part 11 or 11A, and the outer layer part 12 or 12A made of a resin and provided on the surface of the base material part 11 or 11A, in which a predetermined region 121 on the surface side of the outer layer part 12 or 12A is provided with a plurality of irregularly shaped linear portions 13 that are irregularly formed, and are linear portions 13 that are indented toward the base material part 11 or 11A.

The elongated medical instrument according to the present embodiment may be expressed as including, for example, the base material part 11 or 11A, and the outer layer part 12 or 12A made of a resin and provided on the surface of the base material part 11 or 11A, in which a predetermined region 121 on the surface side of the outer layer part 12 or 12A is provided with a plurality of irregularly shaped linear portions 13 that are irregularly formed and originating from the cracks generated in the outer layer part 12 or 12A, and are linear portions 13 that are indented toward the base material part 11 or 11A.

The present embodiment will be described with the recesses being formed in a direction in which the outer diameter becomes smaller than that of the substantially flat surface 121 of the outer layer part 12 or 12A, but an interpretation is possible in which, for example, the irregularly shaped protrusions 14 are formed in a direction in which the outer diameter becomes larger than that of the diameter of the base material part 11.

Hereinafter, the guide wire 1 will be described as an example of an elongated medical instrument. However, the present disclosure can be applied not only to the guide wire 1 but also to the catheter 2. The irregularly shaped recesses can be irregularly formed on the outer layer part provided on the surface of the catheter 2. The present disclosure is applicable to not only a guide wire or the catheter 2, but also to elongated medical members for treatment (such as endoscopes) that are inserted through tubular living tissue such as a blood vessel or the gastrointestinal tract.

### [Example 1]

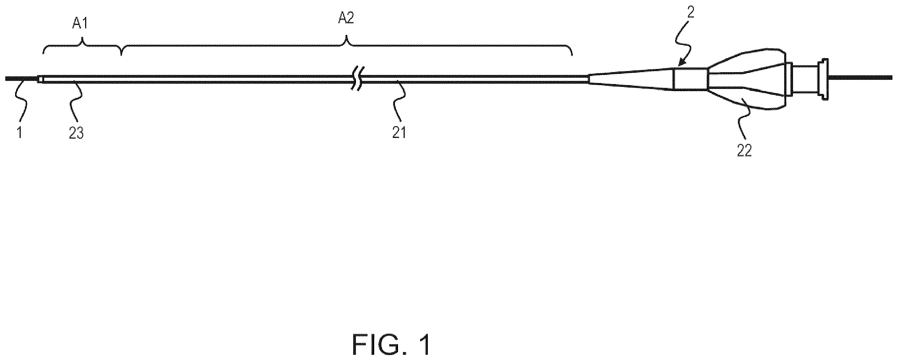

Example 1 will be described using FIG. 1 to FIG. 18. As shown in FIG. 1, a catheter 2 is used, for example, to diagnose or treat a constricted part or an occluded part. The catheter 2 may also be a balloon catheter, a microcatheter, a cardiac catheter, a pulmonary artery catheter, an angiographic catheter, a urethral catheter, a gastrointestinal catheter, or the like.

The catheter 2 includes a catheter shaft 21, and a connector 22 joined to the proximal end side of the catheter shaft 21. The catheter shaft 21, for example, is provided with a coil body, and a resin tube that covers the outside of the coil body (not illustrated). A lumen (not illustrated) through which the guide wire 1 or another catheter can be inserted is formed on the inner peripheral side of the coil body along the longitudinal direction of the coil body.

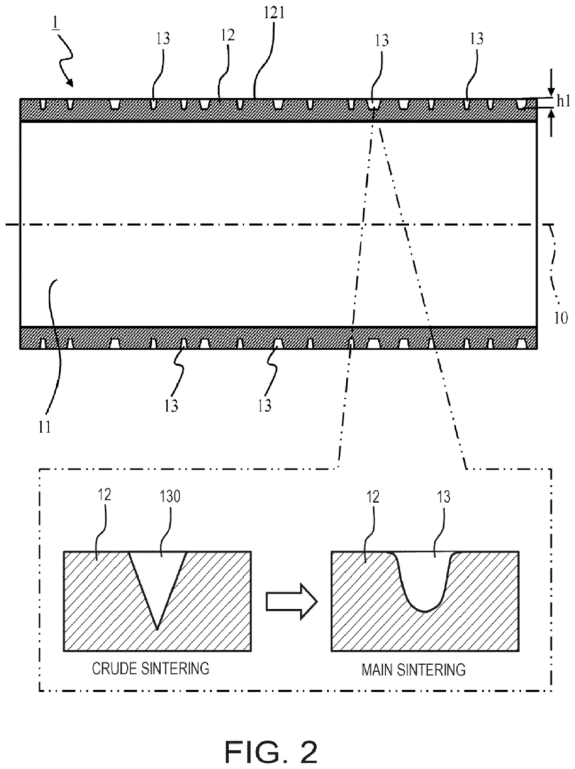

FIG. 2 is a longitudinal sectional view showing enlarged the guide wire 1 according to the present example. The guide wire 1 includes a core material 11, which is an example of the "base material part", an outer layer part 12 provided in close contact so as to cover the surface of the core material 11, and irregularly shaped recesses 13 that are irregularly formed on the surface of the outer layer part 12. The guide wire 1 may include a different resin layer or the like (not illustrated) between the core material 11 and the outer layer part 12. Note that a coil-shaped portion is sometimes provided on the distal end side of the core material 11, but this is not illustrated. FIG. 2 shows enlarged a region of the guide wire 1 in which the coil-shaped portion is not formed. The depth dimension h1 of the recesses 13 is not a constant value, and is variable. However, the depth dimension h1 of the recesses 13 does not exceed the thickness dimension of the outer layer part 12. The reference sign 10 represents the central axis of the guide wire 1.

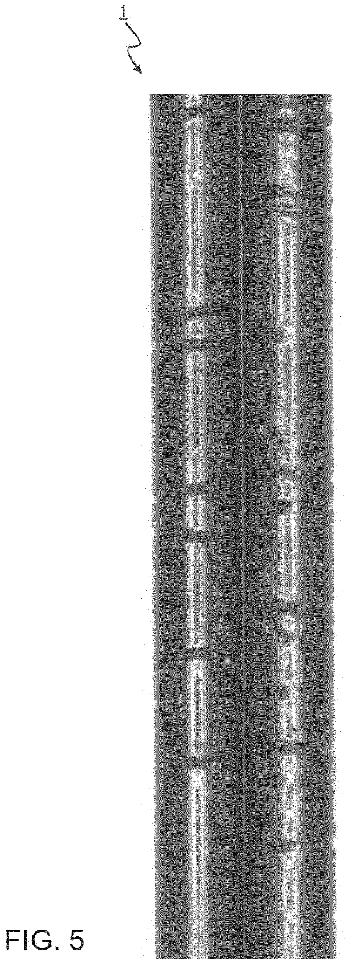

The irregularly shaped recesses 13 are, as shown in FIG. 5, a plurality of wrinkle shaped linear portions formed on the surface of the guide wire 1, and are recessed toward the core material 11 side.

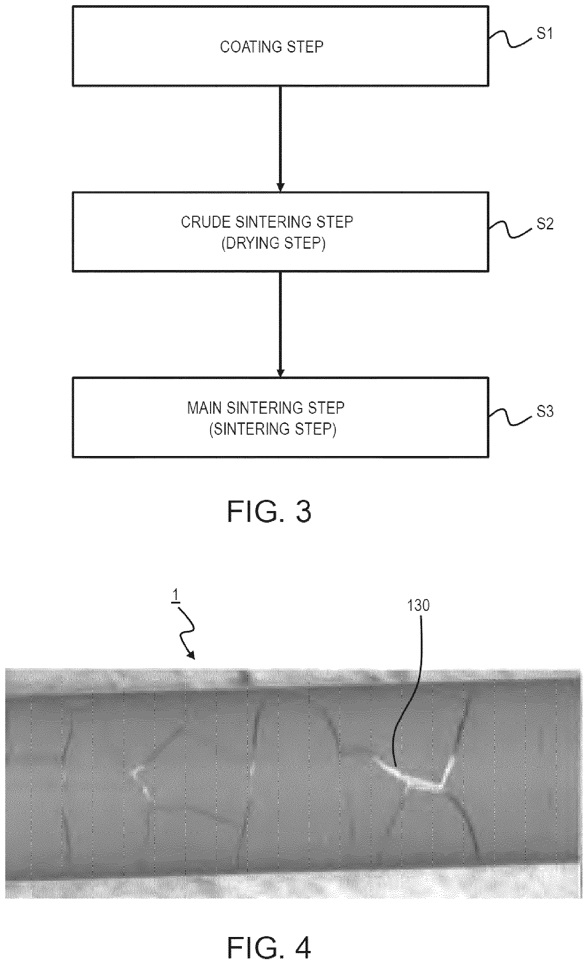

The lower part of FIG. 2 shows a schematic of a transverse cross-section of a recess 13. FIG. 3 shows a method of forming the recesses 13. The description will be continued referring to FIG. 2 and FIG. 3.

In the coating step S1, the core material 11 is dip-coated using a PFA dispersion, which is the material that forms the outer layer part 12. A PFA dispersion is a suspension in which PFA particles (dispersoid) are dispersed in a dispersion medium such as water. The entire circumference may be dip-coated over the entire length of the core material 11, or the entire circumference of a predetermined region of the core material 11 may be dip-coated. The dispersoid is not limited to PFA. Any thermoplastic resin having low frictional resistance can be used as the material of the outer layer part 12.

In the crude sintering step S2, the dip-coated core material 11 is dried by heating at a first temperature TEMP1 for a first time t1 to evaporate the dispersion medium. The crude sintering step can also be called a drying step.

The first temperature TEMP1 may be selected, for example, from a range of temperatures that are at least the evaporation temperature of the dispersion medium, and lower than the melting point of the dispersoid (evaporation temperature of dispersion medium ≤ TEMP1 < melting point of dispersoid). The first time t1 can be set to a predetermined time required for evaporation of the dispersion medium.

By coating the suspension on the core material 11 and heating at the first temperature TEMP1, the dispersion medium evaporates from the surface of the suspension, and the dispersoid solidifies into a film. The suspension state is maintained on the inside. After the surface of the dispersoid has formed a film, the dispersion medium in the suspension successively evaporates from the inner peripheral side near the surface, which causes the volume of the suspension to decrease, and causes portions of the film of the dispersoid at the surface to become recessed. The film of the dispersoid at the surface continues to become recessed while evaporation of the dispersion medium in the suspension continues, which causes cracks 130 to be generated. In the main sintering step S3, the dispersion medium is rapidly evaporated by heating at a higher temperature than the first temperature TEMP1. In the main sintering step S3, the dispersion medium in the suspension is sufficiently evaporated at a level that does not cause detachment of the dispersion medium, and results in the suspension from the crude sintering step S2 that forms the outer layer part 12 to become dried. For example, when a suspension containing ethylene glycol is used as the dispersion medium, the cracks 130 are generated by heating at a first temperature TEMP1 of about 200°C for approximately 2 minutes.

As shown in the lower left of FIG. 2, the crude sintering step S2 results in cracks 130 being generated in the outer layer part 12, which is made of a resin and provided on the surface of the guide wire 1. The lower left of FIG. 2 schematically shows a single crack 130 enlarged, but in reality, as shown in the external view in FIG. 4, a plurality of cracks 130 are formed on the surface of the guide wire 1 after the crude sintering step S2 is completed.

Returning to FIG. 3, in the main sintering step S3, the main sintering is performed by heating the guide wire 1, in which cracks 130 have been generated on the surface, to a second temperature TEMP2 for a second time t2. When the crude sintering step is referred to as the drying step, the main sintering step can also be referred to as the sintering step. The crude sintering step and the main sintering step may be performed consecutively in terms of time.

The second temperature TEMP2 is set higher than the first temperature TEMP1 used during crude sintering (TEMP1 < TEMP2). The second temperature TEMP2 is set to at least the melting point of the dispersoid (here, the melting point of PFA).

The second time t2 can be set to a time that allows the resin above the cracks 130 to melt and flow into the cracks 130, but prevents the cracks 130 from becoming completely buried with the resin that has flowed in. Next, the relationship between the first time t1 and the second time t2 will be described. The first time t1, which is the duration of the crude sintering, may be set to at least the second time t2, which is the duration of the main sintering (t1 ≥ t2). The first time t1 and the second time t2 may be set to the same value or similar values.

As shown in FIG. 2B, the recess 13 is formed as a result of the resin on the surface side of the outer layer part 12 melting and flowing into the crack 130. Because the recess 13 is formed due to the crack 130, it has the same irregular and linear shape as the crack 13. The transverse cross-section of the recess 13 is smoother than the transverse cross-section of the crack 130.

In this way, because the recesses 13 are formed due to the cracks 130 generated in the crude sintering step S2, they are irregularly shaped and linear recesses 13 that are irregularly generated on the surface of the outer layer part 12. A recess 13 that includes either or both of a curved portion and a straight portions can also be referred to as a wrinkle shape.

As mentioned above, FIG. 4 shows enlarged a portion of the surface of the guide wire 1 after completion of the crude sintering step S2. FIG. 5 shows enlarged a portion of the surface of the guide wire 1 after completion of the main sintering step S3. Because FIG. 4 and FIG. 5 show different locations enlarged, and also have different magnifications, the shapes of the cracks 130 and the shapes of the recesses 13 do not correspond, but in reality, as mentioned above, because the recesses 13 are formed as a result of the surrounding resin melting and flowing into the cracks 130, the shapes of the cracks 130 in plan view substantially correspond to the shapes of the recesses 13 in plan view. However, smaller cracks 130 may become filled due to the resin that has flowed in.

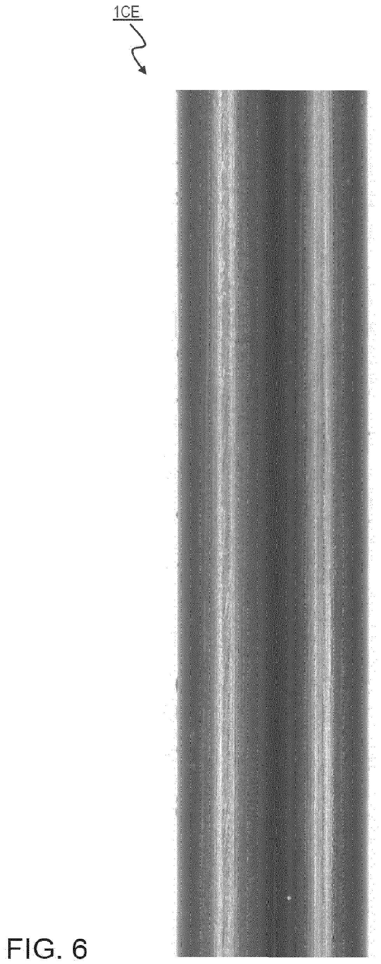

FIG. 6 shows enlarged a portion of a guide wire 1CE according to a comparative example. The surface of the guide wire 1CE according to the comparative example is not formed with irregularly shaped recesses and protrusions 13. If the guide wire 1CE shown in FIG. 6 is observed at a higher magnification than the magnification of the external view in FIG. 5, recesses and protrusions would appear on the surface, though these are not intentionally formed.

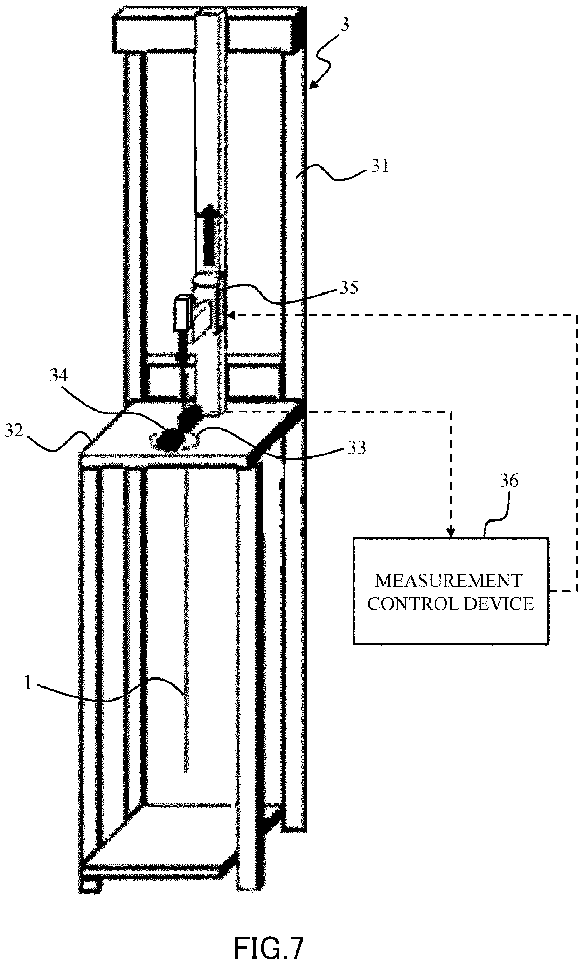

FIG. 7 shows a measurement device 3 that measures the outer diameter dimension of the guide wire 1. The outer diameter measurement device 3 includes, for example, a frame 31, a flat plate portion 32 having an insertion hole 33, an outer diameter measurement instrument 34, a linear actuator 35, and a measurement control device 36. As the outer diameter measurement instrument 34 and the measurement control device 36, it is possible to use, for example, a measurement head LS-9006 and a controller LS-9500 of the ultra-high speed and high precision dimension measurement instrument LS-9000 series from Keyence Corporation.

The frame 31 extends upward from a flat floor surface, and has the flat plate portion 32 attached midway along the vertical direction. The flat plate portion 32 is formed with the insertion hole 33, through which the guide wire 1 is inserted. The outer diameter measurement instrument 34 is disposed around the insertion hole 33. The outer diameter measurement instrument 34, for example, measures the outer diameter dimension of a target object using laser light, and a has a light emitter and a light receiver that are opposingly arranged so as to sandwich the insertion hole 33.

The linear actuator 35 is vertically provided on the flat plate portion 32 in a position slightly away from the insertion hole 33. The linear actuator 35 moves vertically upward at a constant speed according to a control signal from the measurement control device 36 while holding one end portion of the guide wire 1 (the upper end portion of the guide wire 1 in FIG. 7).

As the guide wire 1 is being pulled vertically at a constant speed by the linear actuator 35, the outer diameter measurement instrument 34 measures the outer diameter dimension of the guide wire 1 in real time, and outputs it to the measurement control device 36.

A measurement using the measurement device 3 in FIG. 7 can be performed, for example, using an outer diameter measurement sampling period of 8,000 times/sec, a vertical movement speed of 10 mm/sec, and a measurement length of 30 mm. The external dimension data of the guide wires of the present example and the comparative example obtained by such a measurement will be described below.

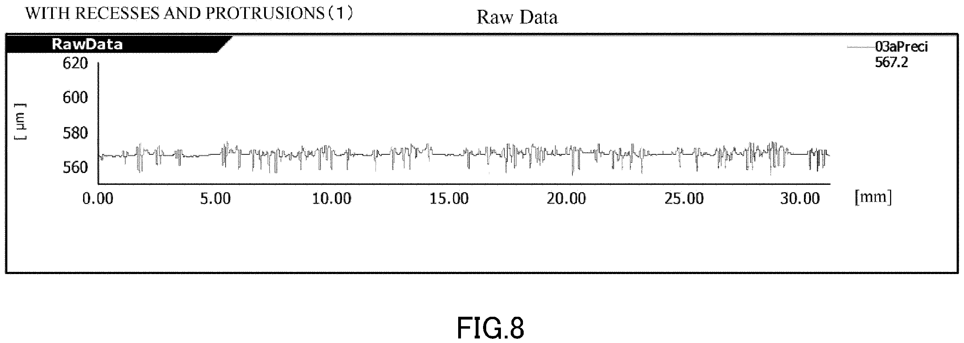

FIG. 8 is a graph showing a result (raw data) of measuring the outer diameter dimension of a guide wire 1 with irregularly shaped recesses and protrusions 13 that have been irregularly formed on the surface of the outer layer part 12 using the outer diameter measurement device 3 described in FIG. 7. The vertical axis represents the outer diameter dimension (pm), and the horizontal axis represents the position in the length direction of the guide wire 1. From the graph in FIG. 8, it can be seen that the surface of the guide wire 1 of the present example has recesses 13 that are irregularly formed with varying concavity.

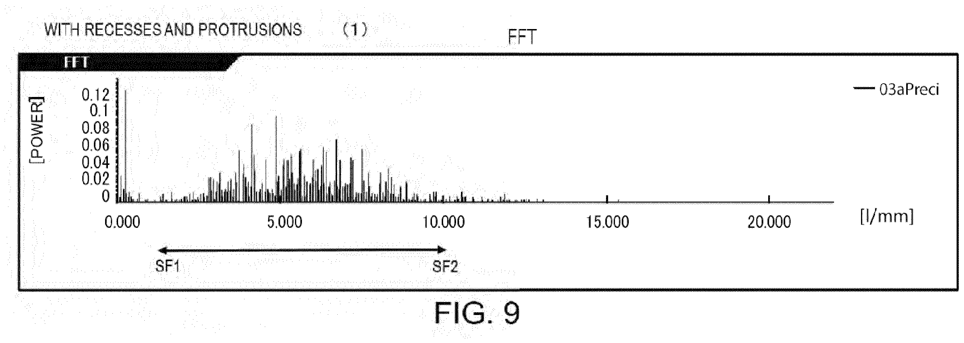

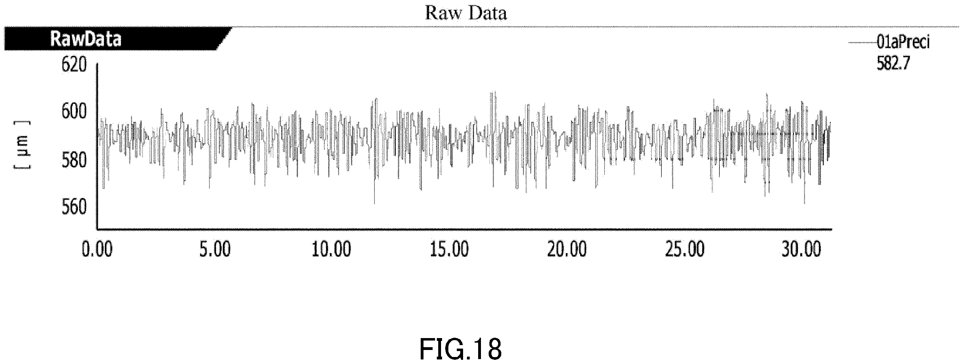

FIG. 9 is a waveform diagram showing the results obtained by an FFT analysis of the measurement data in FIG. 8. The vertical axis represents the power value, and the horizontal axis represents the spatial frequency (l/mm). According to the waveform diagram in FIG. 9, the waveform representing the recesses and protrusions 13 includes spatial frequencies in a range from a lower limit SF1 to an upper limit SF2. The lower limit SF1 is 1 (l/mm) and the upper limit SF2 is 10 (l/mm). That is, it can be determined from the experiment that the sliding resistance is reduced when recesses 13 including spatial frequencies of 1 to 10 (l/mm) are formed. Further, the effects described below in FIG. 18 can also be obtained when recesses and protrusions 13 having various shapes in plan view and in transverse cross-section are irregularly formed on the surface of the guide wire 1 so as to include spatial frequencies from 3 to 10 (l/mm).

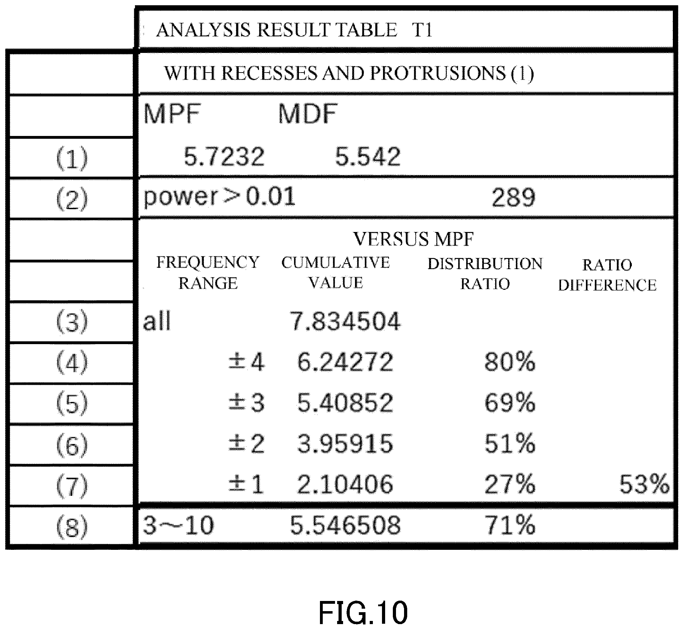

FIG. 10 is a table T1 showing FFT analysis results of the outer diameter dimension of a guide wire 1 having recesses 13.

As displayed in the first row (1) of table T1, in the guide wire 1 of the present example, the MPF is 5.7232 and the MDF is 5.542. As displayed in the second row (2) of table T1, there are 289 data points in which the power value is larger than 0.01.

The third row (3) to the seventh row (7) of table T1 represent the data distribution state when the spatial frequency range is changed with respect to the MPF. As shown in the third row (3), the cumulative power value in the entire spatial frequency range is 7.834504. As shown in the fourth row (4), the cumulative value in the spatial frequency range of MPF plus or minus 4 is 6.24272. The distribution ratio thereof is 80%. As shown in the fifth row (5), the cumulative value in the spatial frequency range of MPF plus or minus 3 is 5.40852. The distribution ratio thereof is 69%. As shown in the sixth row (6), the cumulative value in the spatial frequency range of MPF plus or minus 2 is 3.95915. The distribution ratio thereof is 51%. As shown in the seventh row (7), the cumulative value in the spatial frequency range of MPF plus or minus 1 is 2.10406. The distribution ratio thereof is 27%, and the ratio difference between the distribution ratio for a spatial frequency of plus or minus 4 and the distribution ratio for a spatial frequency of plus or minus 1 is 53%.

As shown in the eighth row (8) of table T1, the cumulative value is 5.546508 in the spatial frequency range of 3 to 10 (l/mm). The distribution ratio thereof is 71%.

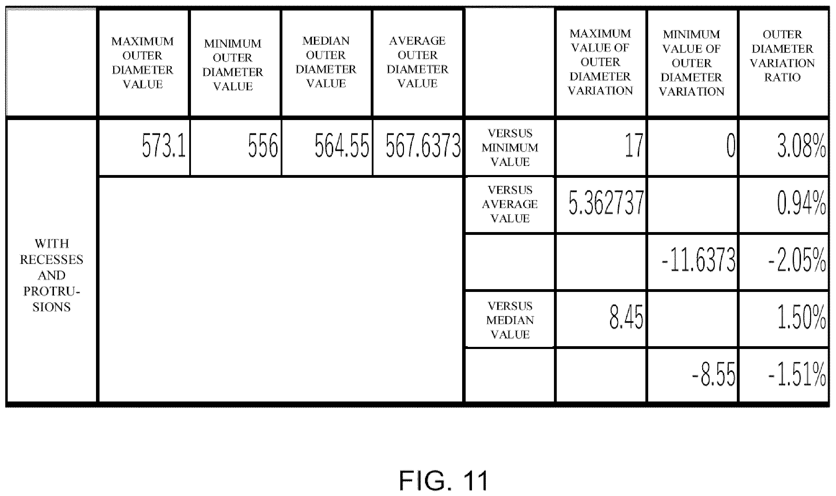

FIG. 11 is a table showing the results of measuring the outer diameter dimension of a guide wire 1 having recesses 13 in the outer layer part 12. The unit is µm. The maximum value of the outer diameter dimension is 573.1, the minimum value of the outer diameter dimension is 556, the median value of the outer diameter dimension is 564.55, the average value of the outer diameter dimension is 567.6373, and the variation value of the outer diameter dimension is 2.452621.

Focusing on the variation of the outer diameter dimension, the maximum value of the outer diameter variation with respect to the minimum value of the outer diameter dimension is 17, the minimum value of the outer diameter variation is 0, and the outer diameter variation ratio is 3.0%. The outer diameter variation value with respect to the average value of the outer diameter dimension is from 5.362737 to -11.6373, and the outer diameter variation ratio is from +0.94% to -2.05%. The outer diameter variation value with respect to the median value of the outer diameter dimension is from 8.45 to -8.55, and the outer diameter variation ratio is from +1.50% to -1.51%.

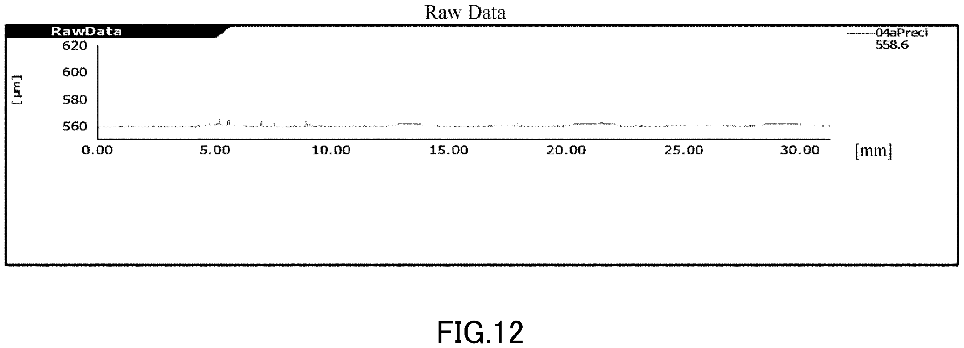

FIG. 12 is a graph of data obtained by measuring the outer diameter dimension of a guide wire 1CE according to a comparative example along the axial direction. The vertical axis represents the outer diameter (pm), and the horizontal axis represents the position in the length direction of the guide wire 1. From the graph in FIG. 12, it can be seen that the surface of the guide wire 1CE of the comparative example is smooth and without recesses and protrusions when compared to the guide wire shown in FIG. 8.

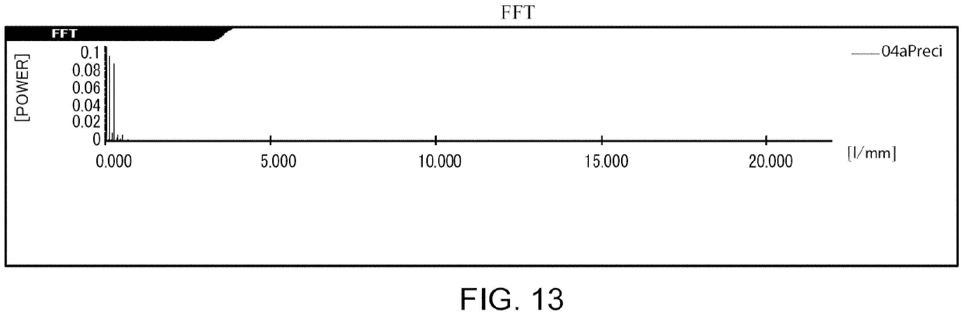

FIG. 13 is a waveform diagram showing the results obtained by an FFT analysis of the measurement data in FIG. 12. The vertical axis represents the power value, and the horizontal axis represents the spatial frequency (l/mm). According to the waveform diagram in FIG. 13, it can be seen that substantially no recesses and protrusions exist on the surface of the guide wire 1CE according to the comparative example.

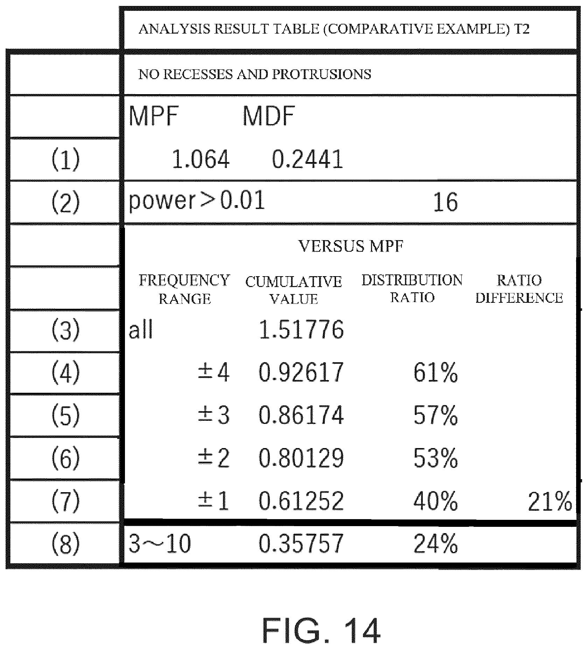

FIG. 14 is a table T2 showing FFT analysis results of the outer diameter dimension of the guide wire 1CE of the comparative example.

As displayed in the first row (1) of table T1, in the guide wire 1CE of the comparative example, the MPF is 1.064 and the MDF is 0.2441. As displayed in the second row (2) of table T2, there are no data points in which the power value is larger than 0.01. The divergence between the MPF value and the MDF value is due to the fact that the power value fluctuates at a level that does not appear in the graph at 1 (l/mm) or more in FIG. 13. Such fluctuations are caused by diameter fluctuations and the like that occur due to the processing of the core material 11 of the guide wire.

The third row (3) to the seventh row (7) of table T2 represent the data distribution state when the spatial frequency range is changed with respect to the MPF. As shown in the third row (3), the cumulative value in the entire spatial frequency range is 1.51776. As shown in the fourth row (4), the cumulative value in the spatial frequency range of MPF plus or minus 4 is 0.926171. The distribution ratio thereof is 61%. As shown in the fifth row (5), the cumulative value in the spatial frequency range of MPF plus or minus 3 is 0.861739. The distribution ratio thereof is 57%. As shown in the sixth row (6), the cumulative value in the spatial frequency range of MPF plus or minus 2 is 0.801288. The distribution ratio thereof is 53%. As shown in the seventh row (7), the cumulative value in the spatial frequency range of MPF plus or minus 1 is 0.612517. The distribution ratio thereof is 40%.

As shown in the eighth row (8) of table T2, the cumulative value is 0.357573 in the spatial frequency range of 3 to 10 (l/mm). The distribution ratio thereof is 24%.

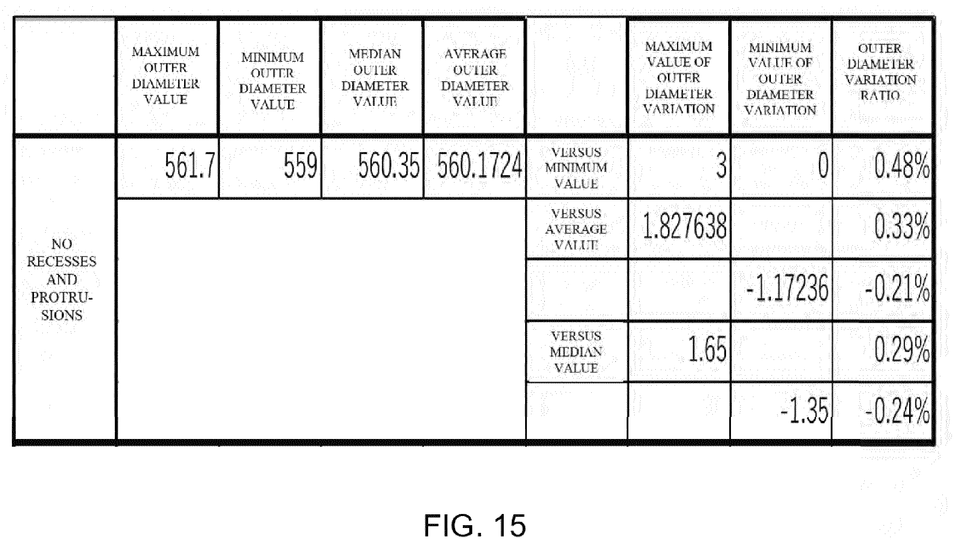

FIG. 15 is a table showing the results of measuring the outer diameter dimension of the guide wire 1CE. The unit is µm. The maximum value of the outer diameter dimension is 561.7, the minimum value of the outer diameter dimension is 559, the median value of the outer diameter dimension is 560.35, the average value of the outer diameter dimension is 560.1724, and the variation value of the outer diameter dimension is 0.662483. Although the description is omitted, the surface of the guide wire 1CE of the comparative example does not have the recesses 13 of the present example, and has almost no variation in the outer diameter because the surface is substantially smooth.

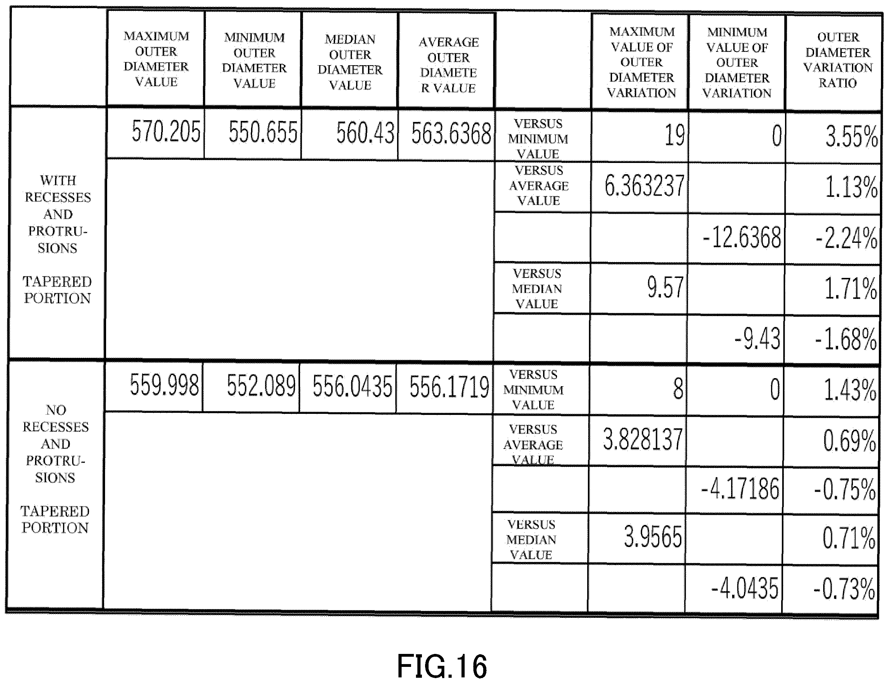

A case where the guide wire 1 has a tapered portion will be described using FIG. 16. FIG. 16 is a table showing the results of measuring the outer diameter dimension of the tapered portion of the guide wire 1.

Although the description is omitted, the guide wire 1 may have a tapered portion in which the diameter gradually decreases toward the distal end side. In the tapered portion of the guide wire 1, because a taper is formed in the core material 11, and recesses 13 are further formed on the surface of the guide wire 1, the change in the outer dimension of the tapered portion is greater than the change in the outer dimension in the non-tapered portion. Therefore, in order to determine the change in the outer dimension due to the formation of the recesses 13 in the tapered portion, it is necessary to obtain a numerical value of the change in the outer dimension that is corrected so as to offset the change in the diameter of the core material 11.

The correction of the change in the outer dimension is performed by approximating the change in the outer diameter of the core material 11 between the distal end and the proximal end of the tapered portion with a linear function, and using the inverse function of the linear function to add or subtract to the outer diameter value according to the distance from the distal end or the proximal end of the tapered portion.

The upper part of FIG. 16 shows analytical values of the measurement data for a case where recesses 13 are irregularly formed on the tapered portion when the core material 11 of the guide wire 1 has a diameter reduction of 8 pm per 10 cm in the longitudinal direction. The lower part of FIG. 16 shows analytical values of the measurement data for a case where recesses 13 are not formed on the tapered portion when the core material 11 of the guide wire 1CE has a diameter reduction of 8 pm per 10 cm in the longitudinal direction. The unit is pm.

The upper part of the table will be described. The maximum value of the outer diameter dimension is 570.205, the minimum value of the outer diameter dimension is 550.655, the median value of the outer diameter dimension is 560.43, the average value of the outer diameter dimension is 563.6368, and the variation value of the outer diameter dimension is 3.269153.

Focusing on the variation of the outer diameter dimension, the maximum value of the outer diameter variation with respect to the minimum value of the outer diameter dimension is 19, the minimum value of the outer diameter variation is 0, and the outer diameter variation ratio is 3.55%. The outer diameter variation value with respect to the average value of the outer diameter dimension is from +6.363237 to -12.6368, and the outer diameter variation ratio is from +1.13% to -2.24%. The outer diameter variation value with respect to the median value of the outer diameter dimension is from +9.57 to -9.43, and the outer diameter variation ratio is from +1.71% to -1.68%.

Therefore, compared to a case where a taper is not present, because the variation in the outer dimension of the tapered portion increases, a correction of the outer dimension of the tapered portion is necessary.

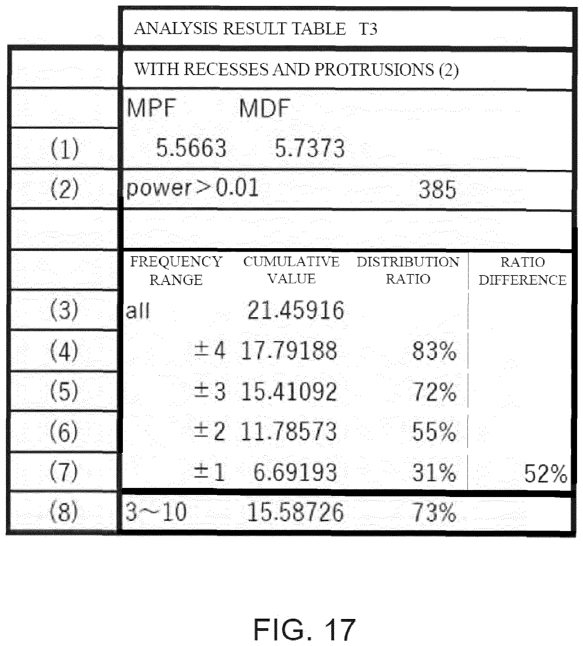

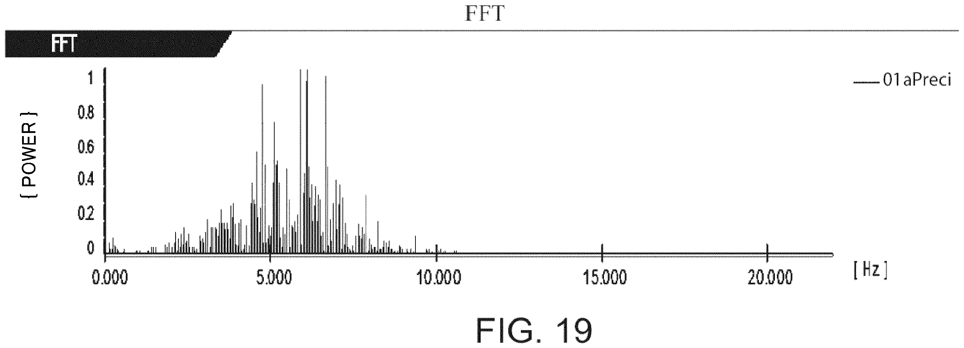

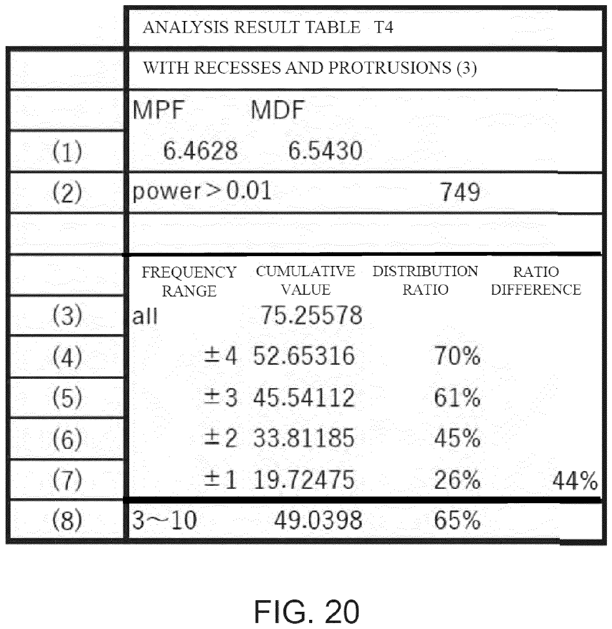

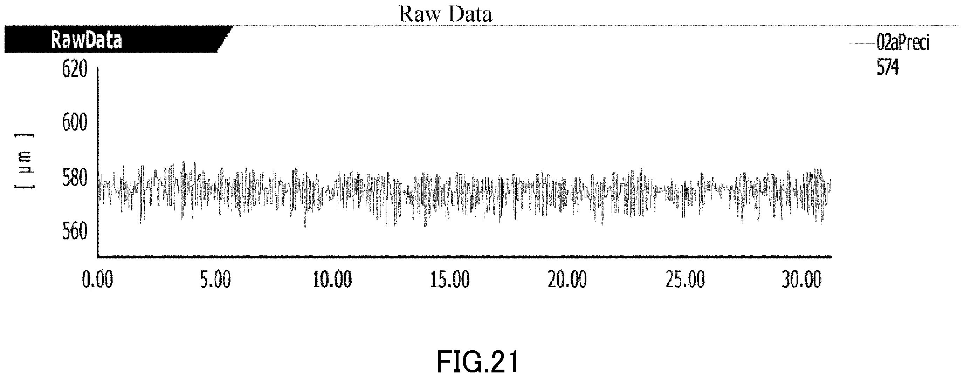

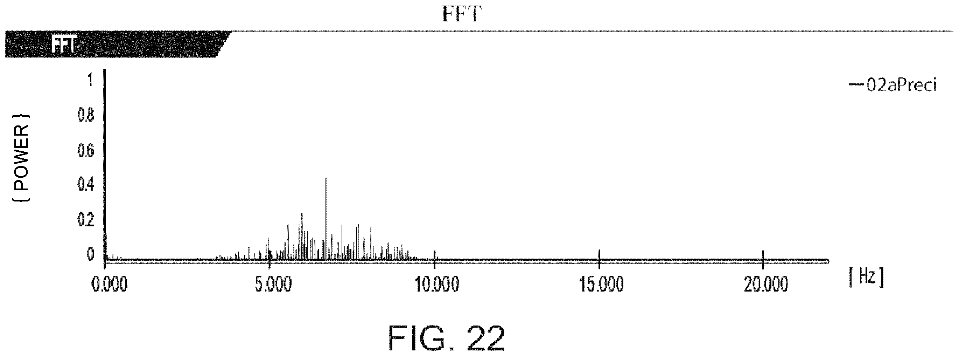

FIG. 17 to FIG. 22 will be described. FIG. 17 is an analysis result table T3 for another case where irregular recesses 13 are formed on the surface of the guide wire 1. FIG. 18 is a graph showing a measurement of an external dimension measurement. FIG. 19 is a graph showing FFT analysis results of the external dimension measurement data. FIG. 20 is an analysis result table T4 for yet another case where irregular recesses 13 are formed on the surface of the guide wire 1. FIG. 21 is a graph showing a measurement of an external dimension measurement. FIG. 22 is a graph showing FFT analysis results of the external dimension measurement data. The analysis result table T3 and the analysis result table T4 are examples where the thickness of the outer layer part 12 made of a resin is formed thicker than in the analysis result table T1 in FIG. 8 to FIG. 10.

As displayed in the first row (1) in tables T1, T3 and T4, the guide wire 1 formed with the recesses 13 has the MPF in the range of 4 to 7, and the MDF also in the range of 4 to 7. As displayed in the second row (2), there are at least 100 data points in which the power value is larger than 0.01.

As displayed in the third row (3) to seventh row (7) of tables T1, T3 and T4, the lower limit of the distribution ratio in a spatial frequency of plus or minus 4 is 66%. The upper limit of the distribution ratio in a spatial frequency of plus or minus 1 is 35%. The lower limit of the distribution ratio in a spatial frequency of plus or minus 4 is the median value of the minimum value of the fourth row (4) of tables T1, T3 and T4 in which recesses and protrusions are formed (70% in T4), and the value (61%) of the fourth row (4) of table T2 in which recesses and protrusions are not formed. The upper limit of the distribution ratio in a spatial frequency of plus or minus 1 is the median value of the maximum value of the seventh row (7) of tables T1, T3 and T4 in which recesses and protrusions are formed (31% in T3), and the value (40%) of the seventh row (7) of table T2 in which recesses and protrusions are not formed. The lower limit of the ratio difference in between the lower limit of the spatial frequency of plus or minus 4 and the upper limit of the spatial frequency of plus or minus 1 in the tables in which recesses and protrusions are formed is 31%.

As shown in the eighth row (8) of tables T1, T3 and T4, it can be seen that in the spatial frequency range of 3 to 10 (l/mm), the irregular recesses 13 according to the present example are formed on the surface of the catheter 1.

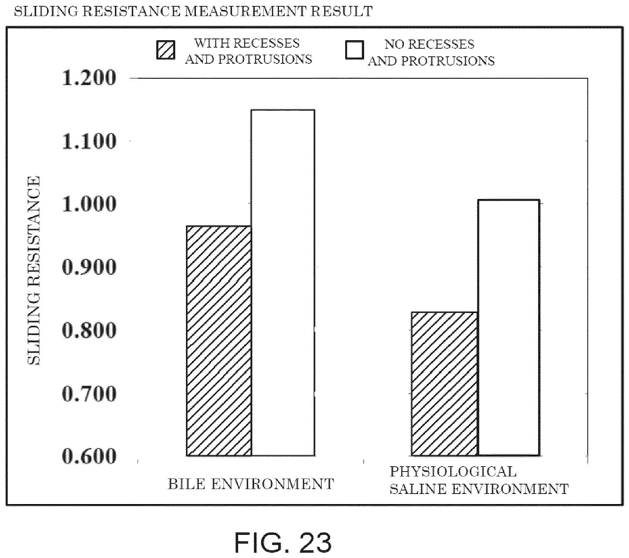

FIG. 23 is a graph showing a comparison of the sliding resistance of the guide wire 1 of the present example, which has the recesses 13 on the surface, and the guide wire 1CE of the comparative example, which does not have recesses on the surface. The vertical axis represents the sliding resistance value. In FIG. 23, a simulated environment was used in which an endoscope was inserted from the oral cavity, through the esophagus and stomach to the duodenum, and a guide wire was inserted through the endoscope. The guide wire 1 and the guide wire 1CE will be compared in a case that simulates introduction of the guide wire into an environment in which, after an endoscope is first inserted into the body in the simulated environment, the inside of the lumen of the endoscope through which the guide wire is inserted is filled with physiological saline, and a case that simulates introduction of the guide wire into an environment in which, after inserting the guide wire into an endoscope, bile flows backward from the distal end of the endoscope and causes the inside of the lumen through which the guide wire is inserted to be filled with bile. The shaded bar graph represents the guide wire 1 of the present example, and the white bar graph represents the guide wire 1CE of the comparative example. In both simulation experiments, it can be seen that the guide wire 1 of the present example having irregular recesses 13 on the surface has a smaller sliding resistance.

According to the present example configured in this manner, the sliding resistance can be reduced by irregularly disposing the recesses 13 on the substantially flat surface 121 of the outer layer part 13. In particular, the shape of the recesses can reduce the sliding resistance by including a shape that appears over a spatial frequency range of 3 to 10 (l/mm).

It is clear from the first row (1) of tables T1 and T3 that the sliding resistance is reduced when the value of at least one of the MDF and MPF is 4 to 7 (l/mm). Similarly, it is clear from the first row (1) of tables T1 and T3 that the sliding resistance is reduced when the value of at least one of the MDF and MPF is 5 (l/mm) or more.

It is clear from the second row (2) of tables T1 and T3 that the sliding resistance is reduced when there are ten or more spatial frequencies with a power value of 0.01 or more.

It is clear from the FFT waveform diagram of FIG. 9 that the sliding resistance is reduced when the spatial frequencies with a power value of 0.01 or more are dispersed over a range of 1 to 10 (l/mm).

It is clear from the seventh row (7) of tables T1 and T3 that the sliding resistance is reduced when the distribution ratio in a range of the average frequency component value plus or minus 1 (l/mm) is less than 35%.

It is clear from the fourth row (4) of tables T1 and T3 that the sliding resistance is reduced when the distribution ratio in a range of the average frequency component value plus or minus 4 (l/mm) is about 80% or more.

It is clear from the ratio difference in the seventh row (7) of tables T1 and T3 that the sliding resistance is reduced when the difference between the distribution ratio of the average frequency component value plus or minus 1 (l/mm) and the distribution ratio of plus or minus 4 (l/mm) is about 50% or more.

It is clear from the eighth row (8) of table T1 that the sliding resistance is reduced when the power spectrum of spatial frequencies 3 to 10 (l/mm) is 50% or more. Similarly, it is clear from the eighth row (8) of table T1 that the sliding resistance is reduced when the power spectrum of spatial frequencies 3 to 10 (l/mm) is 70% or more.

It is clear from the outer diameter variation ratio in FIG. 11 that the sliding resistance is reduced when the outer diameter variation ratio is 3% or more with respect to the minimum outer diameter value.

It is clear from the graphs in FIG. 8, FIG. 18, and FIG. 22 that the sliding resistance is reduced when, in a range of 1 cm in the longitudinal axis direction, there are ten or more locations in which change in the outer diameter is 5 pm or more in a width of less than l/mm.

It is clear from the measurement results when the recesses 13 are formed on the tapered portion shown in the upper part of FIG. 16 that the sliding resistance is reduced when, in a case where the outer layer part 12 includes a tapered portion, the outer diameter variation ratio excluding the change in outer diameter due to the tapered portion is 3% or more with respect to the minimum outer diameter value.

### [Example 2]

Another example will be described below. The present disclosure, for example, focuses on the following viewpoints.

Even in a blood vessel, a highly viscous atheromatous thrombus may occur in a lesion. When an atheromatous thrombus enters the recesses 13 on the surface of the elongated medical instrument, the surface of the recesses of the elongated medical instrument and the atheromatous thrombus come into contact with each other, which generates friction.

The gastrointestinal tract, which represents another tubular tissue inside the body, is filled with digestive fluid which is highly viscous compared to normal blood. When digestive fluid enters the recesses 13 on the surface of the elongated medical instrument, the surface of the recesses 13 of the elongated medical instrument and the digestive fluid come into contact with each other, which generates friction.

The wall of the gastrointestinal tract is highly flexible, and peristalsis reduces the inner diameter of the gastrointestinal tract. Therefore, even a elongated medical instrument having recesses 13 and protrusions 14 on the surface generates friction with the wall of the gastrointestinal tract. When the distance between the protrusions 14 (or the distance between the recesses 13) formed on the elongated medical instrument is long, the surface of the flexible gastrointestinal tract enters and makes contact with the recesses 13 when the elongated medical instrument is inserted into the gastrointestinal tract, which generates friction.

The recesses 13 can be formed on the surface of the elongated medical instrument using a resin suspension containing a fluororesin. In this case, the adhesion between the fluororesin and a component formed on the inner peripheral side may decrease. The component formed on the inner peripheral side of the fluororesin is a metal structure or another type of resin such as urethane. That is, as mentioned above, when a wrinkled outer layer part 12 with irregularly disposed linear recesses 13 is formed on the surface of the base material part 11, the adhesion between the base material part 11 and the outer layer part 12 may decrease.

In view of the viewpoints mentioned above, the present disclosure adopts the following configuration. At least part of the following configuration is also described or substantially described in Example 1.

As described above, the linear recesses 13 are formed due to cracks that are generated when the resin suspension is dried. That is, cracks are irregularly generated when the resin suspension coated on the surface of the elongated medical instrument is dried, and the recesses 13 are formed due to the cracks.

The linear recesses 13 are formed over a range of 90 degrees or more in the circumferential direction of the elongated medical instrument, and are irregularly disposed in the circumferential direction and the longitudinal axis direction. The recesses 13 may be formed around the entire circumference of the elongated medical instrument.

The recesses 13 are formed on the outer layer part 12, which is made of a resin and formed on the surface of the elongated medical instrument, and the depth of the recesses 13 is formed to be plus or minus 7% to plus or minus 25% of the average thickness of the outer layer part 12.

An adhesive layer may also be provided between the outer layer part 12, on which the recesses 13 are formed, and the base material part 11. That is, a fluororesin having an adhesive functional group is formed as an adhesive layer on the inner peripheral side of the resin suspension that forms the recesses 13, and between the metal structure or another type of resin, and the resin suspension is then coated on the outer peripheral side of the adhesive layer. Alternatively, the outer layer part 12, on which the recesses 13 are formed, may be formed by a resin suspension of a fluororesin having an adhesive functional group. Such a configuration makes it possible to improve the adhesion between the outer layer part 12 and the base material part 11.

The present disclosure adopts the configuration described above, which provides the following effects.

When the elongated medical instrument is inserted into a normal blood vessel, because the recesses 13 are formed on the surface of the elongated medical instrument, the area in which the surface of the elongated medical instrument is in contact with the inner wall of the blood vessel is reduced. Therefore, it is possible to achieve low-friction properties when inserting the elongated medical instrument into the blood vessel.

Because the normal low-viscosity blood that is passed before reaching the lesioned blood vessel site is retained in the linear recesses 13, the atheromatous thrombus at the lesioned blood vessel site is prevented from entering the recesses 13, and the friction at the surface of the elongated medical instrument can be reduced.

When the elongated medical instrument is inserted into a tubular tissue such as the gastrointestinal tract, liquid components such as high-viscosity digestive liquid enter the linear recesses 13. In this case, the low-viscosity liquid (water) in the liquid component predominantly seeps into the deep portions (bottom portions) of the recesses 13. Therefore, the high-viscosity liquid components can be prevented from entering the deep portions of the recesses 13, and the friction between the surface of the elongated medical instrument and the tubular tissue can be reduced.

Because the recesses 13 are formed along the circumferential direction and the longitudinal axis direction of the elongated medical instrument, even when a high-viscosity liquid component enters the recesses 13, at least a portion of the liquid component can be transferred in the circumferential direction and/or the longitudinal axis direction of the elongated medical instrument from the entry point. As a result of transferring at least a portion of the high-viscosity liquid component that has entered the recesses 13 to a location that is different from the recesses 13, the friction between the surface of the elongated medical instrument and the tubular tissue can be reduced.

Because the linear recesses 13 are irregularly disposed in the circumferential direction and/or the longitudinal axis direction of the surface of the elongated medical instrument, the possibility that recesses and protrusions on the surface of the flexible tissue of the gastrointestinal or the like matching the recesses 13 and the protrusions 14 formed on the surface of the elongated medical instrument over a given distance can be reduced. Therefore, the contact area between the surface of the tissue and the surface of the elongated medical instrument can be reduced, and the friction at the surface of the elongated medical instrument can be reduced.

In order to provide the adhesive layer made of a fluororesin having an adhesive functional group that bonds with the metal structure or another resin between the outer layer part 12 and the base material part 11, the fluororesin of the adhesive layer and the fluororesin of the outer layer part 12 are compatibly bonded. As a result, the adhesion between base material part 11 composed of a metal structure or another resin and the outer layer part 12 on which the recesses 13 are formed can be improved.

The present disclosure includes a description of the production method of a elongated medical instrument. In the conventional technique, as described in Patent Literature 1 and 2, recesses and protrusions are formed on the surface of the elongated medical instrument by a specially prepared unevenness forming step, such as physical processing or chemical processing. The specially prepared unevenness forming step is a step of pressing a pattern for forming recesses and protrusions while the outer layer resin of the elongated medical instrument remains soft, a step of forming recesses and protrusions by grinding the surface of the outer layer resin after curing the outer layer resin, or a step of forming recesses and protrusions by partially dissolving the surface of the outer layer resin. In the conventional technique, because a dedicated step is specially prepared just for creating the recesses and protrusions on the surface of the elongated medical instrument, the production process of the elongated medical instrument becomes complicated.

In contrast, in the elongated medical instrument according to the present disclosure, a step of drying the resin suspension coated on the base material part causes cracks to be formed in the coating of the dried resin suspension, and then recesses are formed due to the cracks as a result of sintering the coating of the resin suspension. That is, in the production method of a elongated medical instrument according to the present disclosure, the recesses 13 are formed in a series of steps that form the outer layer part 12.

As elongated as cracks can be formed by drying after coating the resin suspension, the resin type used as the solid medium, and the liquid medium are not limited.

In order to reduce the friction when the elongated medical instrument is inserted into the body, the resin type may be a fluororesin or a hydrophilic resin. A perfluoroalkoxyalkane can be used as the fluororesin.

As the liquid medium, water, alcohol, or a mixed solution containing water or alcohol as the main component can be used from the viewpoint of availability, ease of drying, and safety.

When the resin suspension contains a fluororesin, an adhesive layer made of a fluororesin having an adhesive functional group may be formed before coating the resin suspension on the base material part of the elongated medical instrument.

According to the production method of a elongated medical instrument according to the present disclosure, it is not necessary to specially prepare a dedicated step just for forming recesses and protrusions on the surface of the elongated medical instrument, and therefore, the production process of the elongated medical instrument can be prevented from becoming complicated. According to the production method of a elongated medical instrument according to the present disclosure, an adhesive layer is formed between the outer layer part 12 and the base material part 11, and therefore, it is possible to improve the adhesion between the base material part 11 and the outer layer part 12 formed of a fluororesin from the resin suspension.

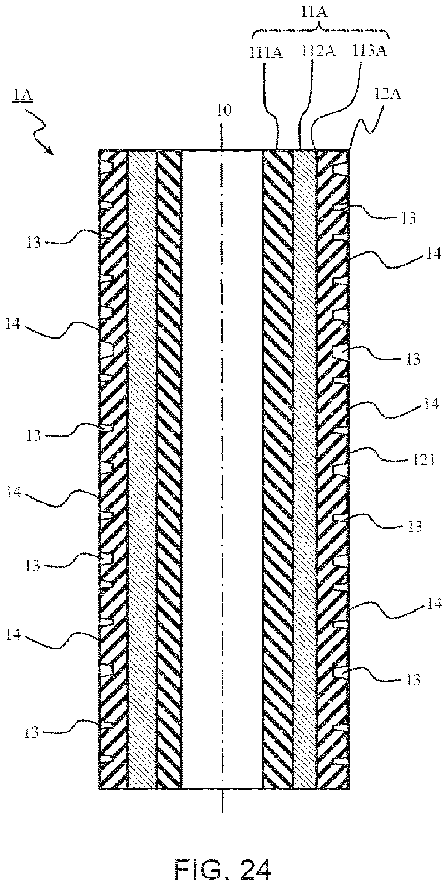

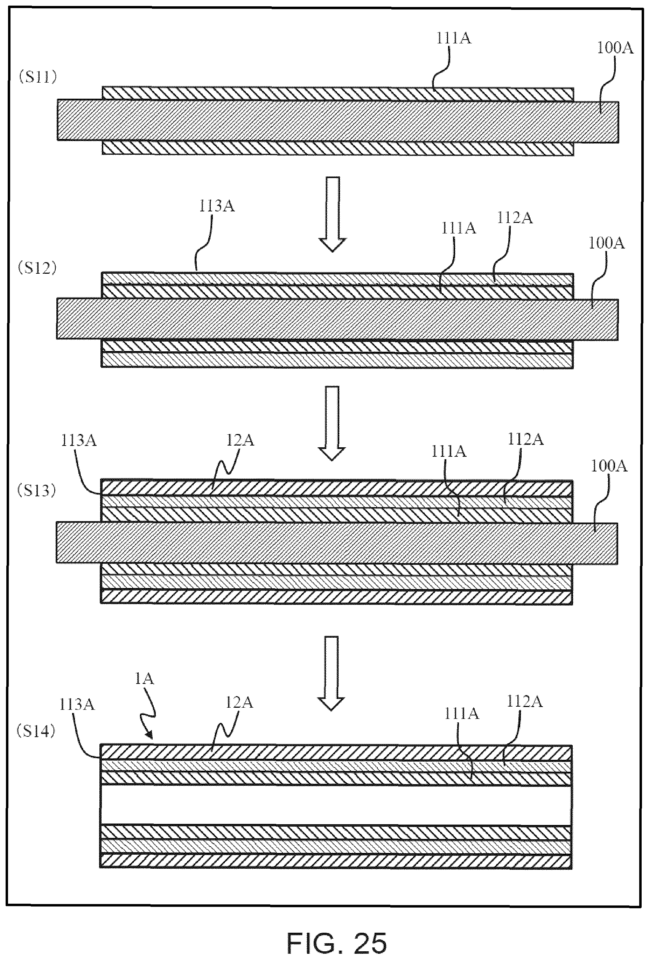

Example 2 will be described using FIG. 24 and FIG. 25. In Example 2 and Example 3 described below, a medical tube will be described as an example of the elongated medical instrument. The medical tube 1A can be used, for example, as a sheath for a digestive basket. The medical tube 1A can also be used for other catheters.

The medical tube 1A includes a base material part 11A and an outer layer part 12A. The base material part 11Aincludes a tubular body 111A, and an intermediate resin layer 112A provided on the outer peripheral side of the tubular body 111A. An adhesive layer 113A for improving the adhesion with the inner peripheral surface of the outer layer part 12A is provided on the outer peripheral surface of the intermediate resin layer 112A. The adhesive layer 113Amay be thought of as a portion of the base material part 11A.

The recesses 13 and protrusions 14 are formed on the surface of the outer layer part 12A. The recesses 13 and the protrusions 14 can be thought of as being oppositely related. That is, when the outer layer part 12A is imagined as a flat surface, the locations where the recesses 13 are not present can be thought of as the protrusions 14. The following description focuses on the recesses 13. The recesses 13 are formed as short lines that extend along the circumferential direction and the longitudinal axis direction of the medical tube 1A, and are irregularly disposed. The actual appearance will be described below.

FIG. 25 shows a production method of the medical tube 1A. In a first step S11, a PFA tube 111A is provided by extrusion molding as a "tubular body" on the outer peripheral surface of a core metal 100A. The PFA tube 111Ais subjected to surface treatment. The surface treatment of the PFA tube 111A is, for example, processing for increasing the adhesion between the PFA tube 111A and the intermediate resin layer 112A. In a second step S12, an intermediate resin layer 112Amade of PTFE is formed by coating a PTFE solution on the outer peripheral surface of the PFA tube 111A, or is provided by extrusion molding of a PTFE tube on the outer periphery of the PFA tube 111A. An adhesive layer 113Amay also be provided on the surface of the intermediate resin layer 112A.

The material of the intermediate resin layer 112A is not limited to polytetrafluoroethylene (PTFE). The intermediate resin layer 112Amay be at least one type of resin selected from a group comprising PTFE, tetrafluoroethylene-perfluoroalkyl vinyl ether copolymer (PFA), tetrafluoroethylene-hexafluoropropylene copolymer (FEP), nylon, and urethane.

When nylon or polyurethane or the like is used as the intermediate resin layer 112A, a fluororesin having an adhesive functional group can be used as an adhesive layer in order to improve the adhesion between the nylon or polyurethane and PFA. That is, a fluororesin having an adhesive functional group (also referred to as adhesive fluororesin) can be used as the adhesive layer 113A between the nylon or polyurethane and PFA.

In a third step S13, a PFA solution is coated on the outer peripheral surface of the intermediate resin layer 112A (or when the adhesive layer 113A is provided on the outside of the intermediate resin layer 112A, on the outer peripheral surface of the adhesive layer 113A), and drying and sintering is carried out in the same manner described in Example 1.

In a fourth step S14, the core metal 100A is pulled out to form the medical tube 1A.

### [Example 3]

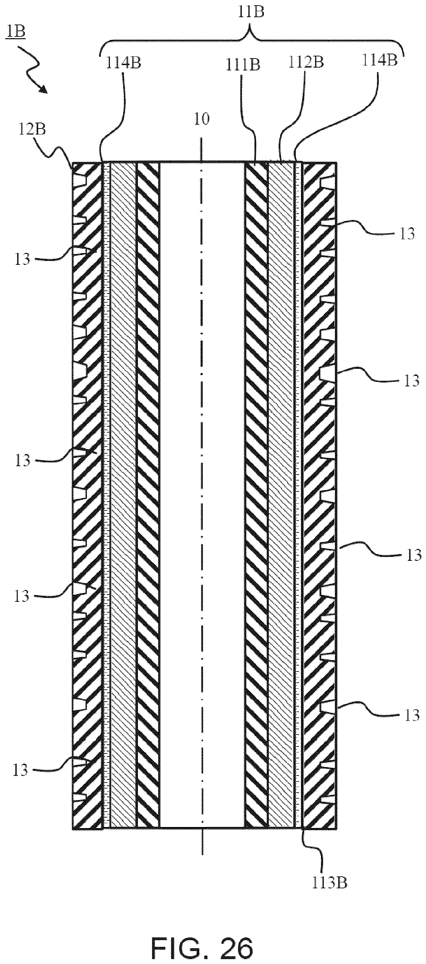

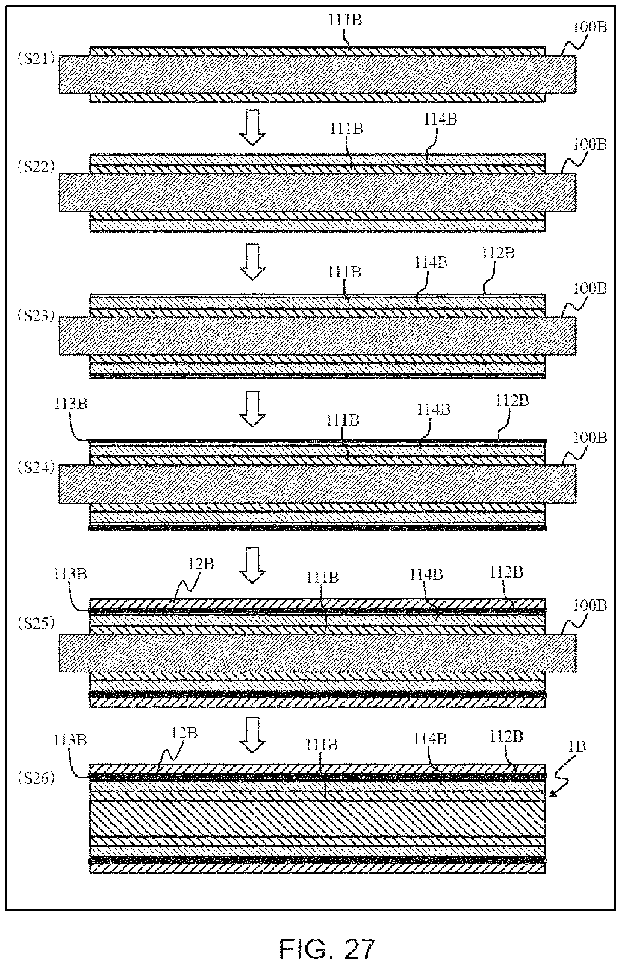

Example 3 will be described using FIG. 26 and FIG. 27. The medical tube 1B includes a base material part 11B and an outer layer part 12B. The base material part 11B includes a tubular body 111B, and an intermediate resin layer 112B provided on the outer peripheral side of the tubular body 111B. An adhesive layer 113B for improving the adhesion with the inner peripheral surface of the outer layer part 12B is provided on the outer peripheral surface of the intermediate resin layer 112B. The adhesive layer 113B may be thought of as a portion of the base material part 11B. The intermediate resin layer 112B may include a reinforcing body 114B.

The recesses 13 and protrusions 14 are formed on the surface of the outer layer part 12B. The recesses 13 are formed as short lines that extend along the circumferential direction and the longitudinal axis direction of the medical tube 1B, and are irregularly disposed. The actual appearance will be described below.

FIG. 27 shows a production method of the medical tube 1B. In a first step S21, a PFA tube 111B is provided by extrusion molding as a "tubular body" on the outer peripheral surface of a core metal 100B. The PFA tube 111B is subjected to surface treatment.

In a second step S22, a reinforcing body 114B is provided on the outer peripheral side of the PFA tube 111B.

In a third step S23, an intermediate resin layer 112B made of PTFE is formed by coating the reinforcing body 114B with a PTFE solution such that it becomes buried.

In a fourth step S24, an adhesive layer 113B is provided on the surface of the intermediate resin layer 112B.

In a fifth step S25, a PFA solution is coated on the outer peripheral surface of the adhesive layer 113B, and by performing drying and sintering in the same manner as described in Example 1, an outer layer part 12B is formed on which the recesses 13 and the recesses 14 are formed.

In a sixth step S26, the core metal 100B is pulled out to form the medical tube 1B.

### [Example 4]

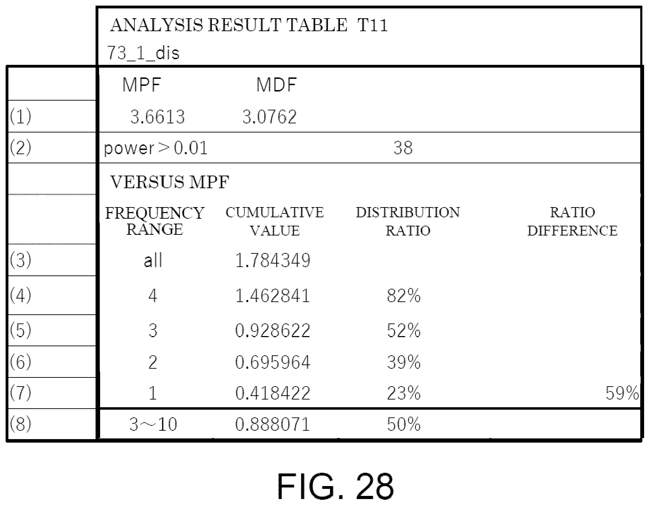

Example 4 will be described using FIG. 28 to FIG. 41. In Example 4 and Example 5 described below, a guide wire will be described as an example of the elongated medical instrument.

FIG. 28 is a table T11 obtained by an FFT analysis of measurement data for the far side of a guide wire according to Example 4.

As displayed in the first row (1) of table T11, on the far side of the guide wire, the MPF is 3.6613 and the MDF is 3.0762. As displayed in the second row (2) of table T11, there are 38 data points in which the power value is larger than 0.01.

The third row (3) to the seventh row (7) of table T11 represent the data distribution state when the spatial frequency range is changed with respect to the MPF. As shown in the third row (3), the cumulative power value in the entire spatial frequency range is 1.784349. As shown in the fourth row (4), the cumulative value in the spatial frequency range of MPF plus or minus 4 is 1.462841. The distribution ratio thereof is 82%. As shown in the fifth row (5), the cumulative value in the spatial frequency range of MPF plus or minus 3 is 0.928622. The distribution ratio thereof is 52%. As shown in the sixth row (6), the cumulative value in the spatial frequency range of MPF plus or minus 2 is 0.695964. The distribution ratio thereof is 39%. As shown in the seventh row (7), the cumulative value in the spatial frequency range of MPF plus or minus 1 is 0.418422. The distribution ratio thereof is 23%, and the ratio difference between the distribution ratio for a spatial frequency of plus or minus 4 and the distribution ratio for a spatial frequency of plus or minus 1 is 59%.

As shown in the eighth row (8) of table T11, the cumulative value is 0.888071 in the spatial frequency range of 3 to 10 (l/mm). The distribution ratio thereof is 50%.

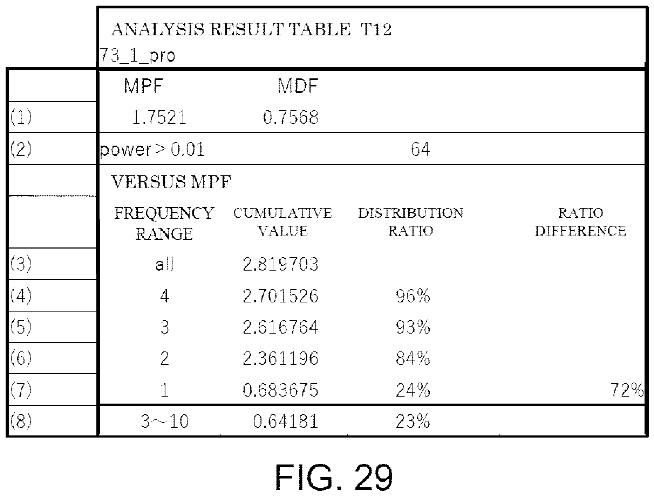

FIG. 29 is a table T12 obtained by an FFT analysis of measurement data for the near side of a guide wire according to Example 4.

As displayed in the first row (1) of table T12, on the near side of the guide wire, the MPF is 1.7521 and the MDF is 0.7568. As displayed in the second row (2) of table T12, there are 64 data points in which the power value is larger than 0.01.

The third row (3) to the seventh row (7) of table T12 represent the data distribution state when the spatial frequency range is changed with respect to the MPF. As shown in the third row (3), the cumulative power value in the entire spatial frequency range is 2.819703. As shown in the fourth row (4), the cumulative value in the spatial frequency range of MPF plus or minus 4 is 2.701526. The distribution ratio thereof is 96%. As shown in the fifth row (5), the cumulative value in the spatial frequency range of MPF plus or minus 3 is 2.616764. The distribution ratio thereof is 93%. As shown in the sixth row (6), the cumulative value in the spatial frequency range of MPF plus or minus 2 is 2.361196. The distribution ratio thereof is 84%. As shown in the seventh row (7), the cumulative value in the spatial frequency range of MPF plus or minus 1 is 0.683675. The distribution ratio thereof is 24%, and the ratio difference between the distribution ratio for a spatial frequency of plus or minus 4 and the distribution ratio for a spatial frequency of plus or minus 1 is 72%.

As shown in the eighth row (8) of table T21, the cumulative value is 0.64181 in the spatial frequency range of 3 to 10 (l/mm). The distribution ratio thereof is 23%.

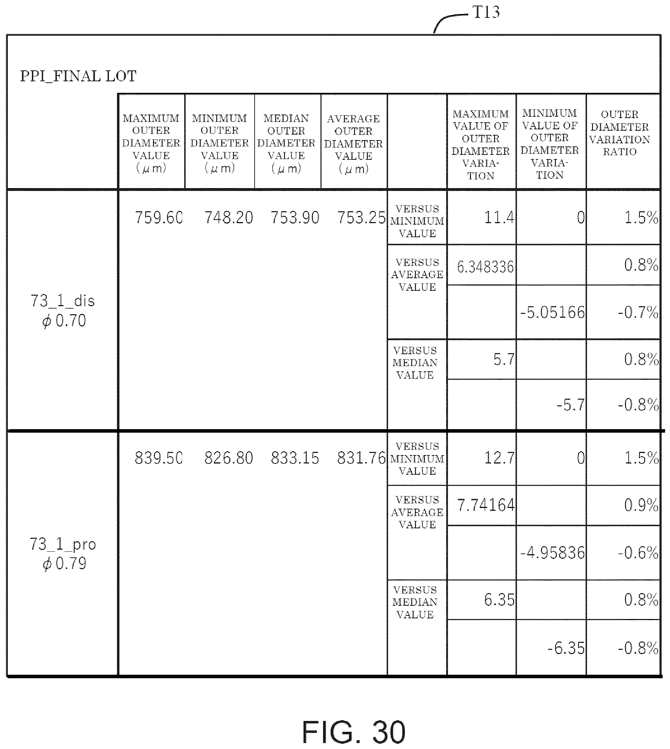

FIG. 30 is a table T13 showing measurement data of a guide wire. The diameter of the guide wire on the far side is 0.70 mm, and the diameter on the near side is 0.79 mm.

The upper part of table T13 shows measurement data for the far side, and the lower part of table T13 shows measurement data for the near side. The measurement data includes the maximum value of the outer diameter, the minimum value of the outer diameter, the median value of the outer diameter, the average value of the outer diameter (all in units of µm), the maximum value of the outer diameter variation, the minimum value of the outer diameter variation, and the outer diameter variation ratio.

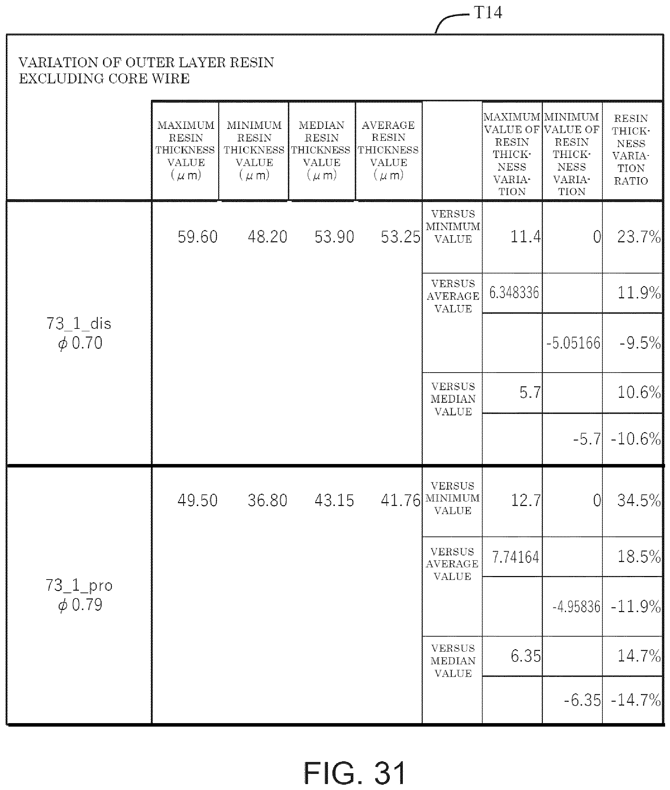

FIG. 31 is a table T14 showing data measuring the variation of the outer layer part 12 (labeled as outer layer resin) excluding the core wire from the guide wire.

The guide wire includes a core wire as the base material part 11, and an outer layer resin as the outer layer part 12, which is provided on the outside of the core wire. The irregularly disposed recesses 13 are formed on the surface of the outer layer resin. The core wire itself does not affect the formation of the recesses 13. Therefore, in the present example, the depth level of the recesses 13 is described with respect to the thickness of the outer layer resin layer that is capable of forming the recesses 13.

The upper part of table T14 shows measurement data for the outer layer resin on the far side, and the lower part of table T14 shows measurement data for the outer layer resin on the near side. The measurement data includes the maximum value of the outer layer resin thickness, the minimum value of the outer layer resin thickness, the median value of the outer layer resin thickness, the average value of the outer layer resin thickness (all in units of µm), the maximum value of the outer layer resin thickness variation, the minimum value of the outer layer resin thickness variation, and the outer layer resin thickness variation ratio. In the diagram, the outer layer resin thickness is labeled as the resin thickness.

From table T14, it can be seen that the maximum depth of the recesses (versus the minimum value) with respect to the entire outer layer resin thickness is in a range of 17 to 85%. If recesses having a depth that is 100% of the outer layer resin thickness are formed, detachment of the outer layer resin will occur starting at the recesses. Therefore, recesses 13 are formed that do not reach 100% of the outer layer resin thickness.

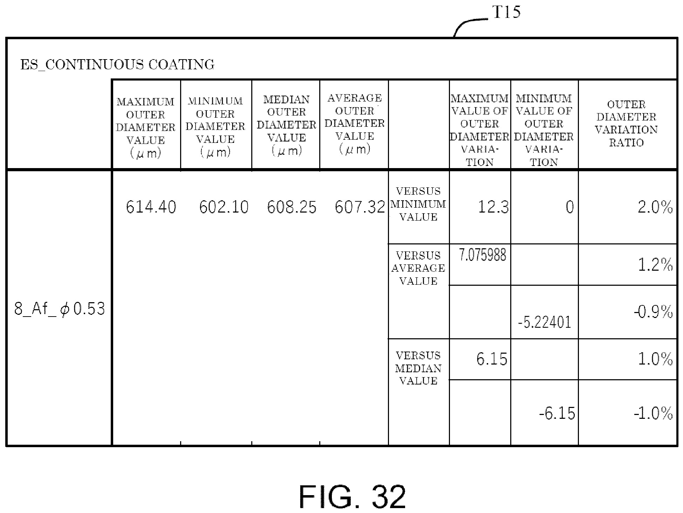

FIG. 32 is a table T15 showing measurement data for another form of a guide wire. The diameter of the guide wire is 0.53 mm. The measurement data recorded in table T15 includes the maximum value of the outer diameter, the minimum value of the outer diameter, the median value of the outer diameter, the average value of the outer diameter (all in units of µm), the maximum value of the outer diameter variation, the minimum value of the outer diameter variation, and the outer diameter variation ratio.

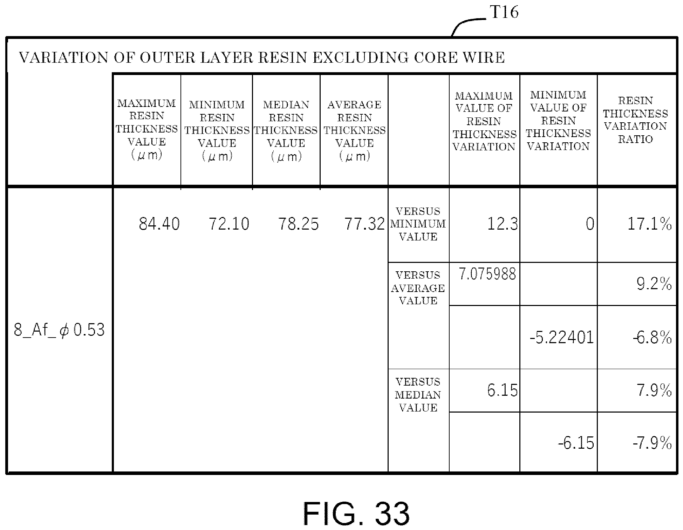

FIG. 33 is a table T16 showing data measuring only the variation of the outer layer part (labeled as outer layer resin) of the guide wire described in T15. The measurement data of the outer layer resin thickness includes the maximum value of the outer layer resin thickness, the minimum value of the outer layer resin thickness, the median value of the outer layer resin thickness, the average value of the outer layer resin thickness (all in units of µm), the maximum value of the outer layer resin thickness variation, the minimum value of the outer layer resin thickness variation, and the outer layer resin thickness variation ratio.

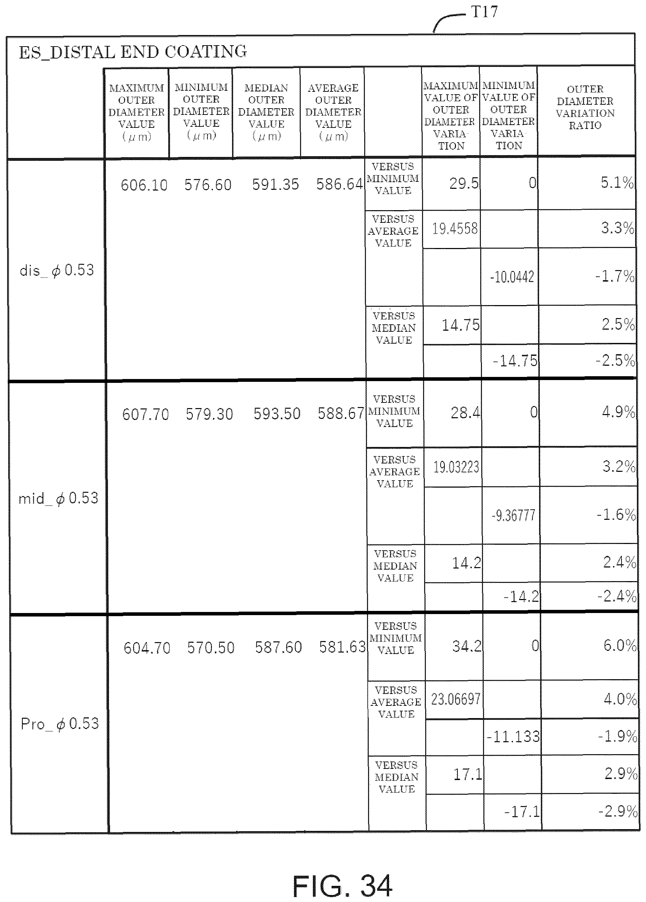

FIG. 34 is a table T17 showing measurement data for yet another form of a guide wire. The diameter of the guide wire is 0.53 mm, and the diameter of the far side, the middle portion, and the near side are all the same. The measurement data recorded in table T17 includes the maximum value of the outer diameter, the minimum value of the outer diameter, the median value of the outer diameter, the average value of the outer diameter (all in units of µm), the maximum value of the outer diameter variation, the minimum value of the outer diameter variation, and the outer diameter variation ratio.

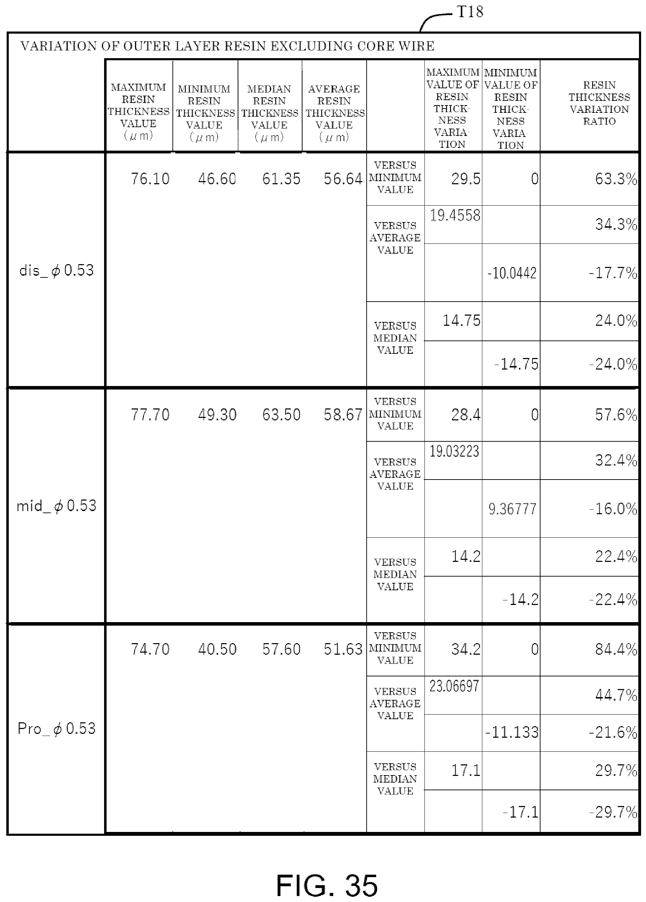

FIG. 35 is a table T18 showing data measuring only the variation of the outer layer part (labeled as outer layer resin) of the guide wire described in T17. The measurement data of the outer layer resin thickness includes the maximum value of the outer layer resin thickness, the minimum value of the outer layer resin thickness, the median value of the outer layer resin thickness, the average value of the outer layer resin thickness (all in units of µm), the maximum value of the outer layer resin thickness variation, the minimum value of the outer layer resin thickness variation, and the outer layer resin thickness variation ratio.

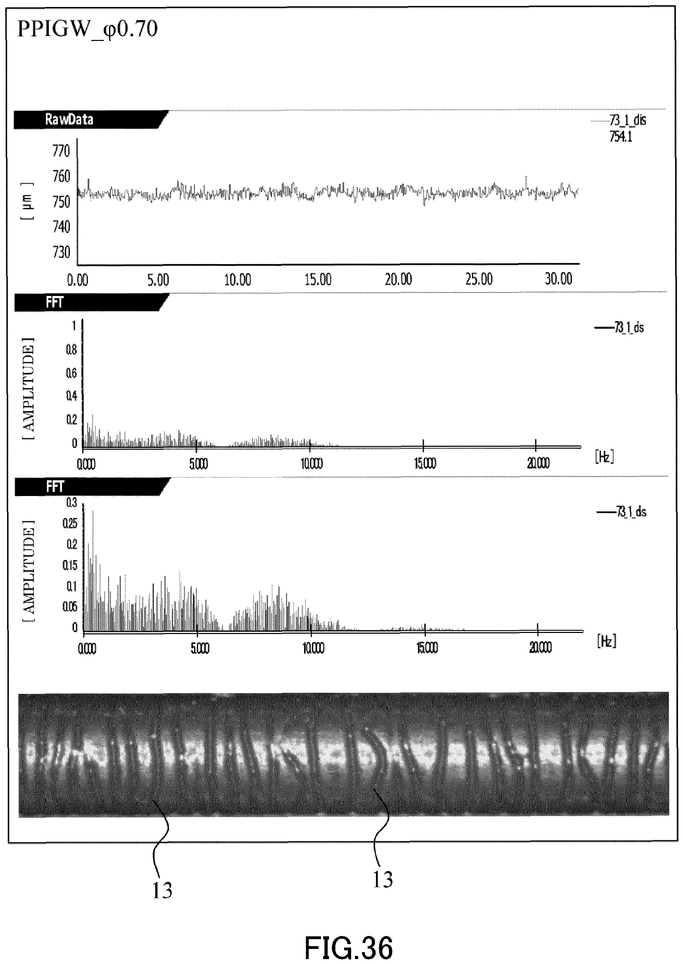

FIG. 36 shows measurement data and an enlarged external view of the far side of the guide wire corresponding to FIG. 30. FIGS. 36A-36D show, in order from the top, the original outer diameter dimension data (the unit of the horizontal axis is mm, the unit of the vertical axis is µm), waveform data obtained by an FFT analysis of the original outer diameter dimension data (the unit of the horizontal axis is Hz, the unit of the vertical axis is amplitude), waveform data obtained by enlarging the amplitude of the waveform data of the FFT analysis, and the appearance.

As indicated by the appearance, a plurality of recesses 13 are formed on the surface of the guide wire over a range of 90 degrees or more in the circumferential direction. Focusing on the shape, it can be seen that the recesses 13 are a mixture of a plurality of shapes such as a linear shape, an S-shape, an inverted S-shape, a meandering shape, and a curved shape.

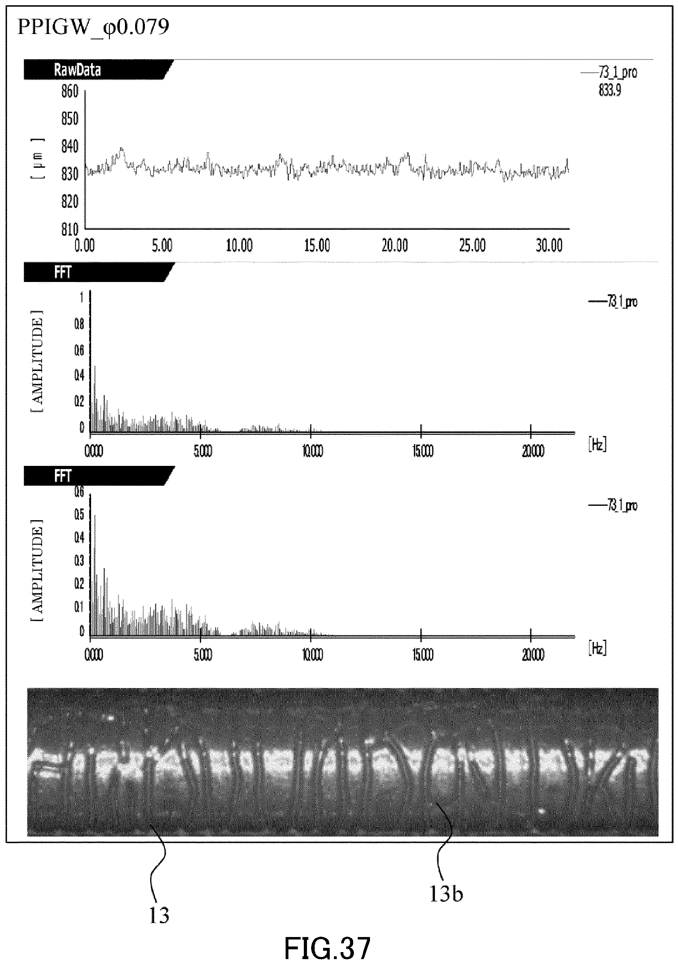

FIG. 37 is measurement data and an enlarged external view of the near side of the guide wire corresponding to FIG. 30. Many of the recesses 13 are independent of each other. However, some recesses 13b are observed in which adjacent recesses are connected to each other.

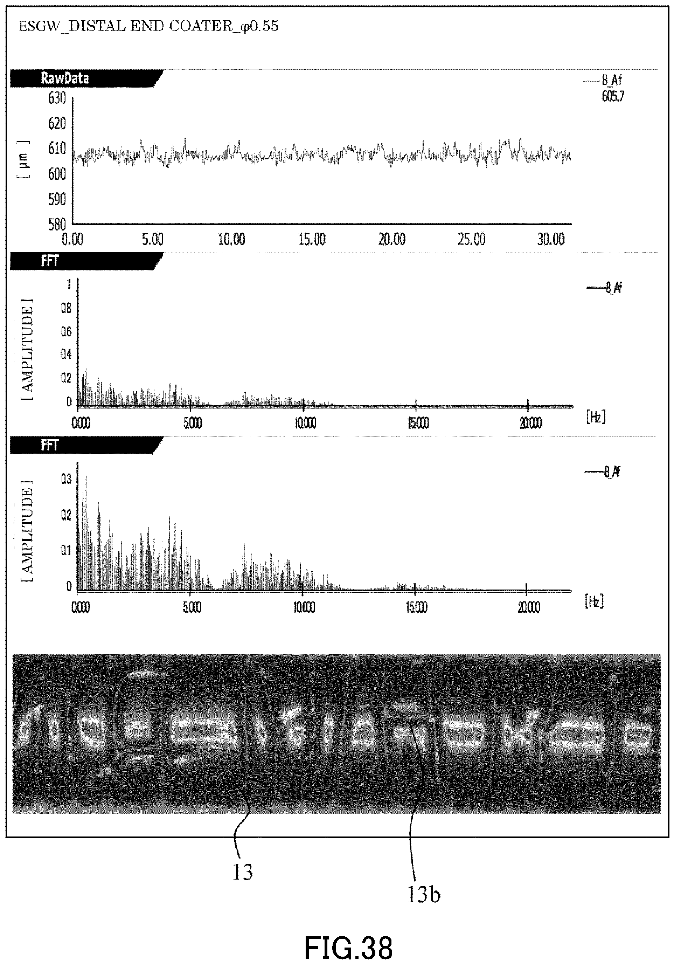

FIG. 38 is measurement data and an enlarged view of the guide wire corresponding to FIG. 32.

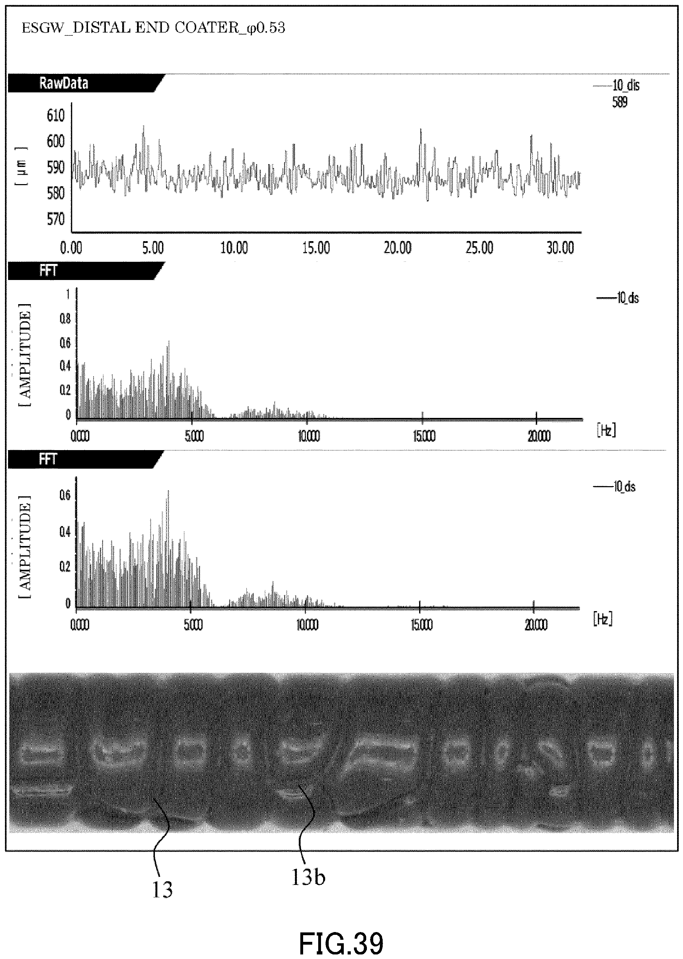

FIG. 39 is measurement data and an enlarged view of the guide wire in FIG. 34.

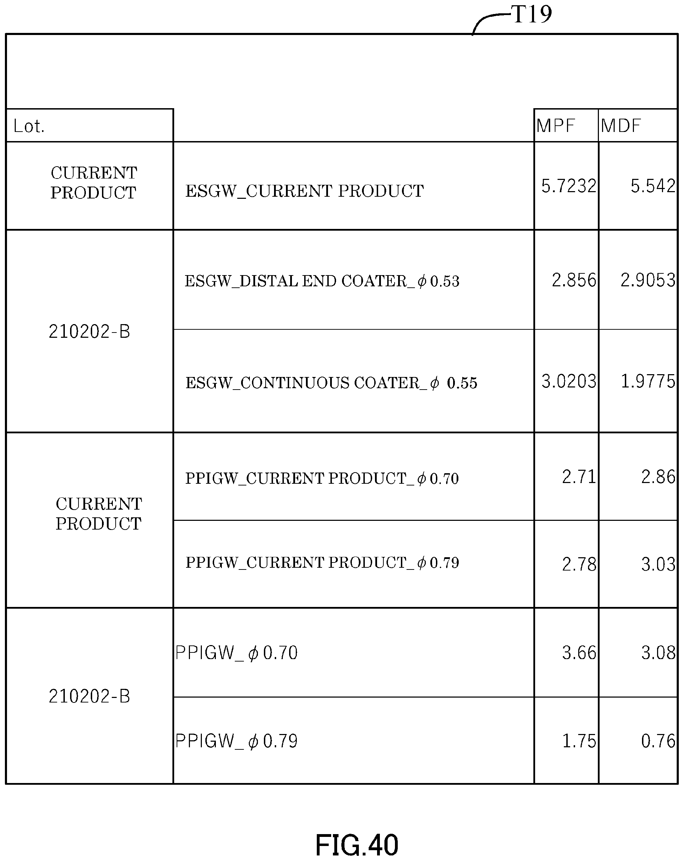

FIG. 40 is a table T19 showing the MPF and MDF values analyzed for the plurality of guide wires described in FIG. 36 to FIG. 39. In FIG. 40, the "current product" and the "distal end coater" are obtained by partially or completely coating a core wire that has been cut to a predetermined length for use as a guide wire with a resin suspension by a method such as dipping, and then performing drying and sintering. It is possible to form the outer layer and the recesses 13 on a portion of the core wire of the "current product" and the "distal end coater". For example, after attaching an outer layer coil to the distal end of the guide wire, it is possible to form the outer layer and the recesses 13 only in a proximal region of the guide wire, and excluding the region in which the outer layer coil is attached. The "continuous coater" is obtained by coating a resin suspension on a core wire that has been wound into a roll, and then winding it back into a roll after performing drying and sintering. The "continuous coater" has the outer layer and the recesses 13 formed on the entire core wire, which has a fixed diameter, and as a result of cutting to a predetermined length during production of the guide wire, and performing diameter reduction processing of the distal end portion by grinding and the like, the outer layer is removed in the region subjected to the diameter reduction processing. Whichever production method is adopted in the production of the guide wire, the outer layer and the recesses 13 are formed in a region proximal to the region in which the outer layer coil is attached.

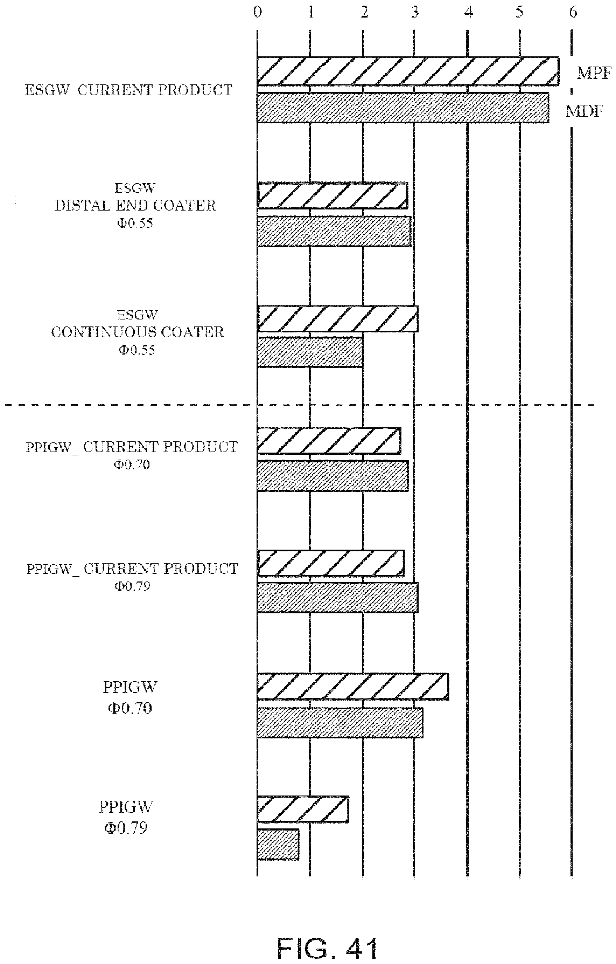

FIG. 41 is an explanatory diagram showing the table T19 in FIG. 40 as a graph.

### [Example 5]

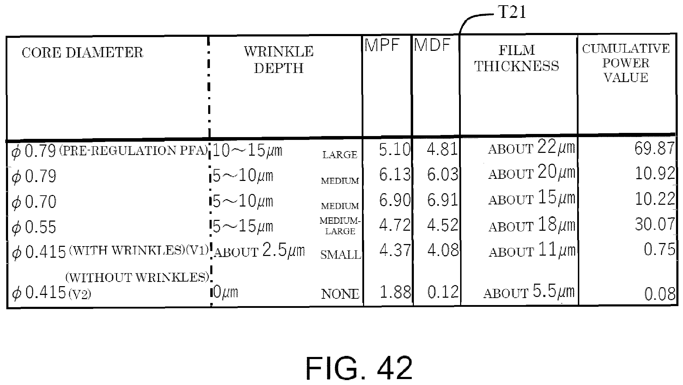

Example 5 will be described using FIG. 42 to FIG. 59. In the present example, a case will be described where recesses are formed on the surface of a guide wire which is thinner than the guide wire described so far.

FIG. 42 is a table T21 obtained by analyzing measurement data for a guide wire according to Example 5. The table T21 shows measurement data for a guide wire with a diameter of 0.415 mm in addition to the measurement data for guide wires with a diameter of 0.79 mm, a diameter of 0.70 mm, and a diameter of 0.55 mm. Further, for the guide wire with a diameter of 0.415 mm, table T21 lists a case where wrinkles (recesses 13) are formed, and a case where wrinkles are not formed.

In Example 5, the occurrence of recesses 13 is measured by changing the speed V (referred to as the coating speed or the outer layer resin forming speed) in which the resin that forms the outer layer part 12 is coated on the surface of the guide wire.

The measurement data in table T21 includes the depth of the recesses 13 (labeled as "wrinkle depth"), MPF, MDF, film thickness, and cumulative power value. Here, the wrinkle depth is defined as half of the difference between the recesses and protrusions in the outer diameter data. Comparing the wrinkle depth, MPF and MDF of the guide wire having a diameter of 0.55 mm to the wrinkle depth, MPF and MDF of the guide wire having a diameter of 0.415 and formed with wrinkles, there is no large change in the MPF and MDF of the guide wire having a diameter of 0.55 mm and the MPF and MDF of the guide wire having a diameter of 0.415 mm. Therefore, the wrinkle depth can be considered to have no effect on the MPF and MDF.

Comparing the cumulative power values of the guide wires of each diameter, the cumulative power value of the guide wire not formed with wrinkles is much lower than the cumulative power value of the other guide wires. Therefore, it can be considered that the wrinkle depth can be estimated to some extent from the cumulative power value.

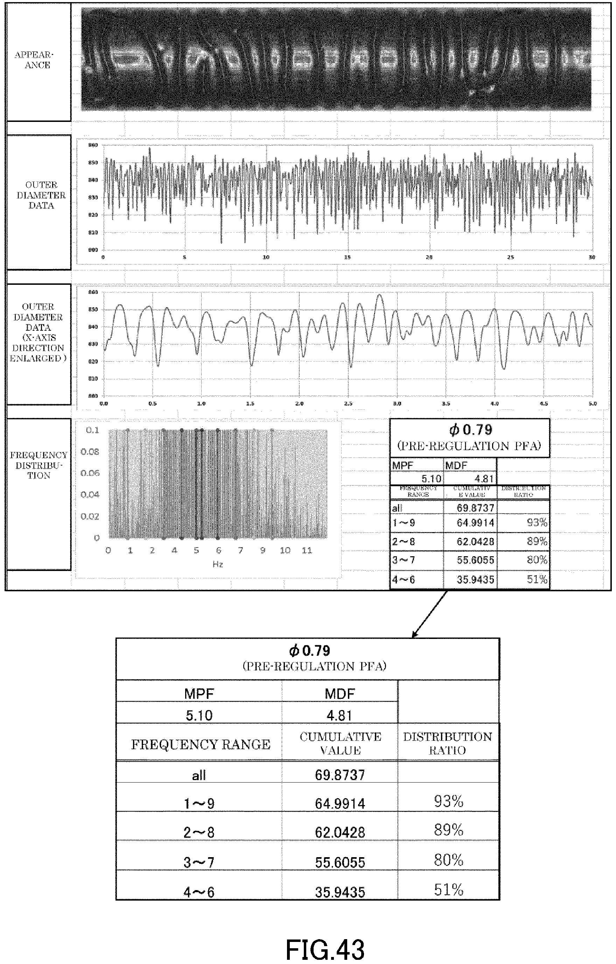

FIG. 43 is an explanatory diagram showing an enlarged external view and outer diameter data of a guide wire having a diameter of 0.79 mm. FIG. 43 shows, in order from the top, the appearance of the guide wire, a graph of the outer diameter data of the guide wire, a graph of the outer diameter data enlarged in the X-axis direction, a frequency distribution, and a table containing MPF and MDF values. The table containing the MPF and MDF values has substantially the same structure as tables T11 and T12 described in FIG. 28 and FIG. 29. The table is shown enlarged in the lower part of FIG. 43.

The vertical axis of the graph of the outer diameter data is in pm, and the maximum of the scale is "860". The horizontal axis of the graph of the outer diameter data is in mm, and the maximum of the scale is "30". The maximum of the scale in the graph with the X-axis direction enlarged is "5.0". The MPF of the guide wire is 5.10, and the MDF is 4.81.

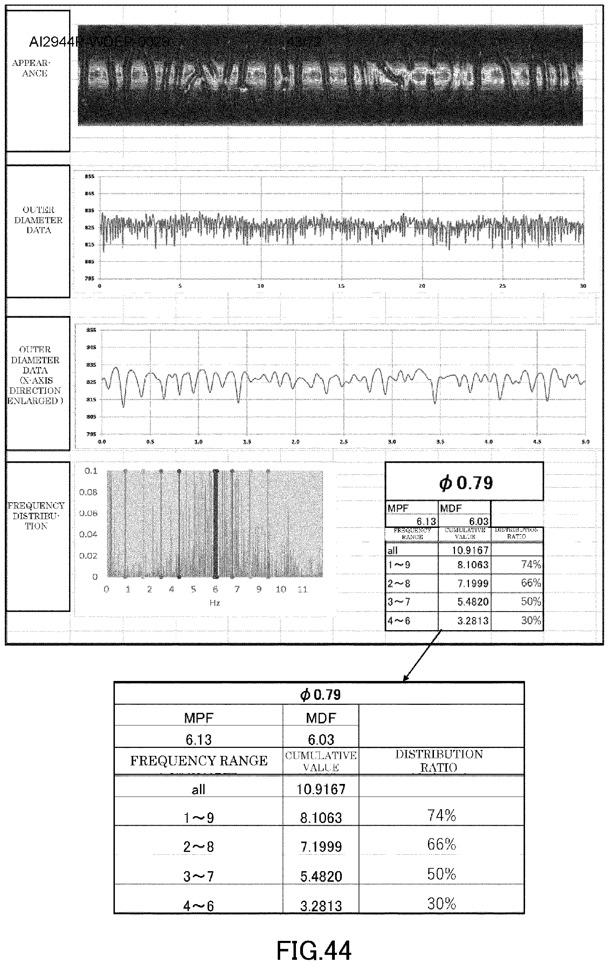

FIG. 44 shows an enlarged external view and outer diameter data and the like of another guide wire having a diameter of 0.79 mm. The MPF of the guide wire is 6.13, and the MDF is 6.03.

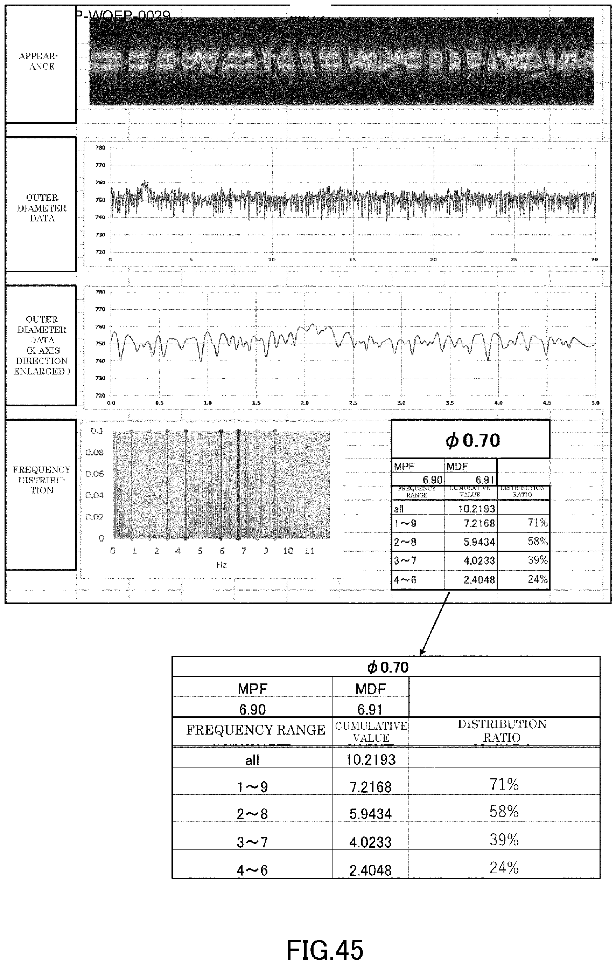

FIG. 45 is an enlarged external view and data of a guide wire having a diameter of 0.70 mm. The MPF of the guide wire is 6.90, and the MDF is 6.91.

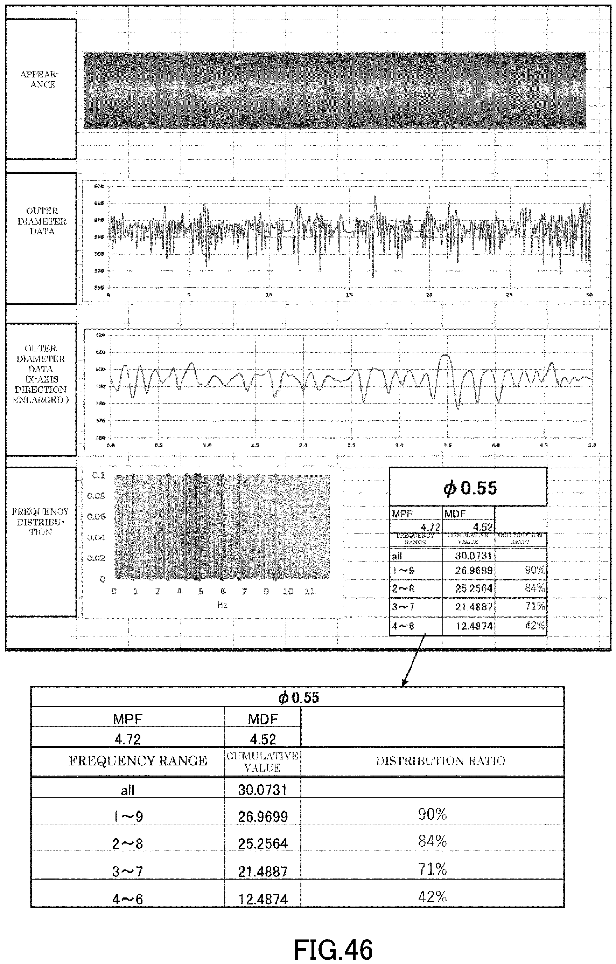

FIG. 46 is an enlarged external view and data of a guide wire having a diameter of 0.55 mm. The MPF of the guide wire is 4.72, and the MDF is 4.52.

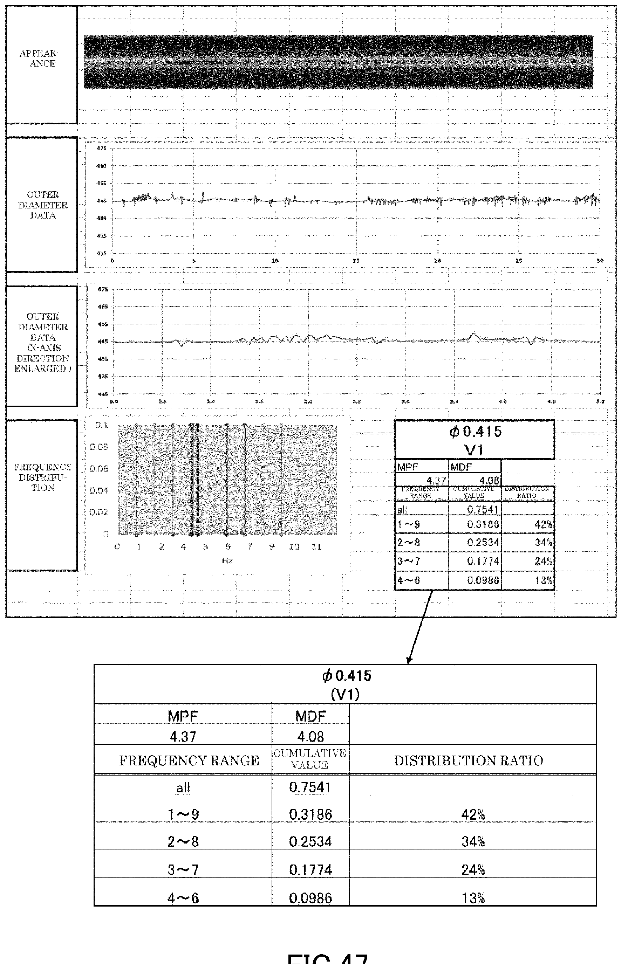

FIG. 47 is an enlarged external view and data for a case where a guide wire having a diameter of 0.415 mm is resin-coated at a coating speed V1. The MPF of the guide wire is 4.37, and the MDF is 4.08.

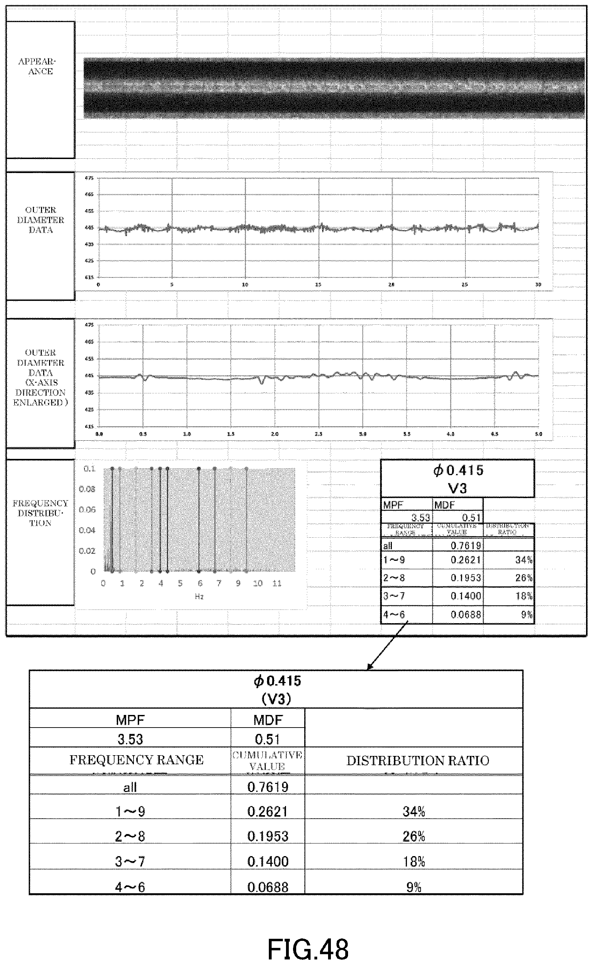

FIG. 48 is an enlarged external view and data for a case where a guide wire having a diameter of 0.415 mm is resin-coated at a coating speed V3. The MPF of the guide wire is 3.53, and the MDF is 0.51.

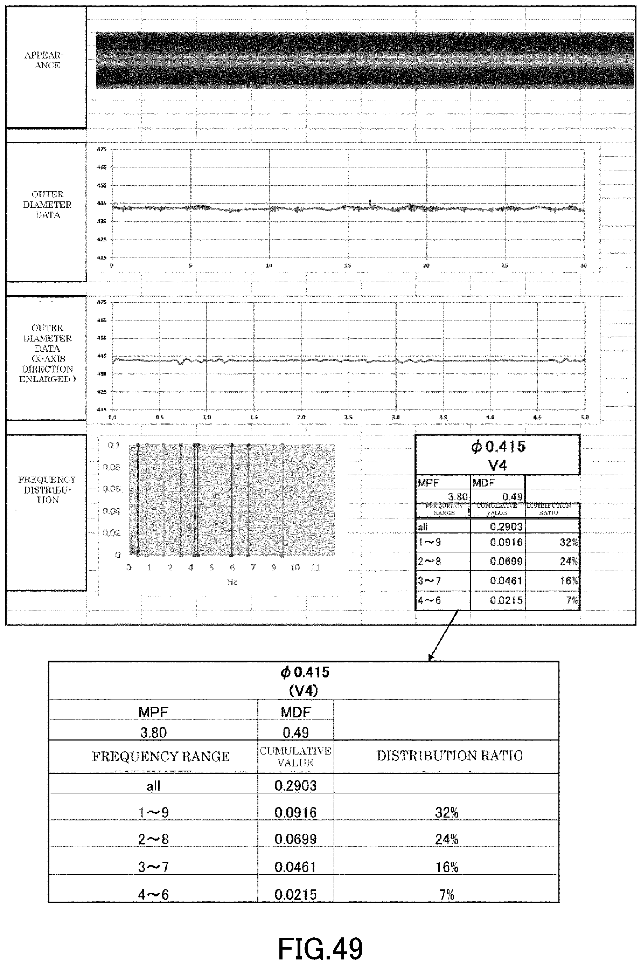

FIG. 49 is an enlarged external view and data for a case where a guide wire having a diameter of 0.415 mm is resin-coated at a coating speed V4. The MPF of the guide wire is 3.80, and the MDF is 0.49.

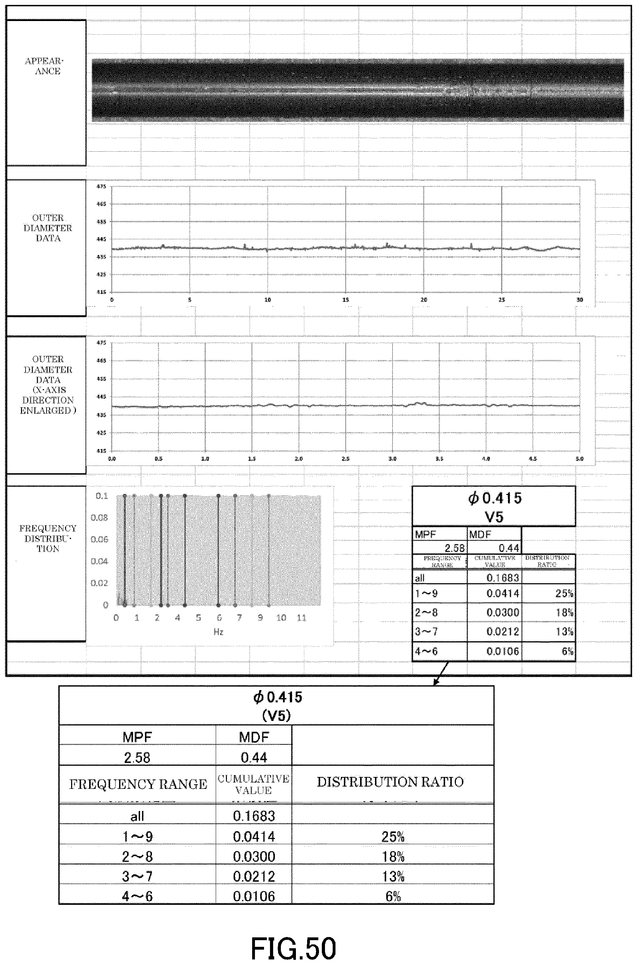

FIG. 50 is an enlarged external view and data for a case where a guide wire having a diameter of 0.415 mm is resin-coated at a coating speed V5. The MPF of the guide wire is 2.58, and the MDF is 0.44.

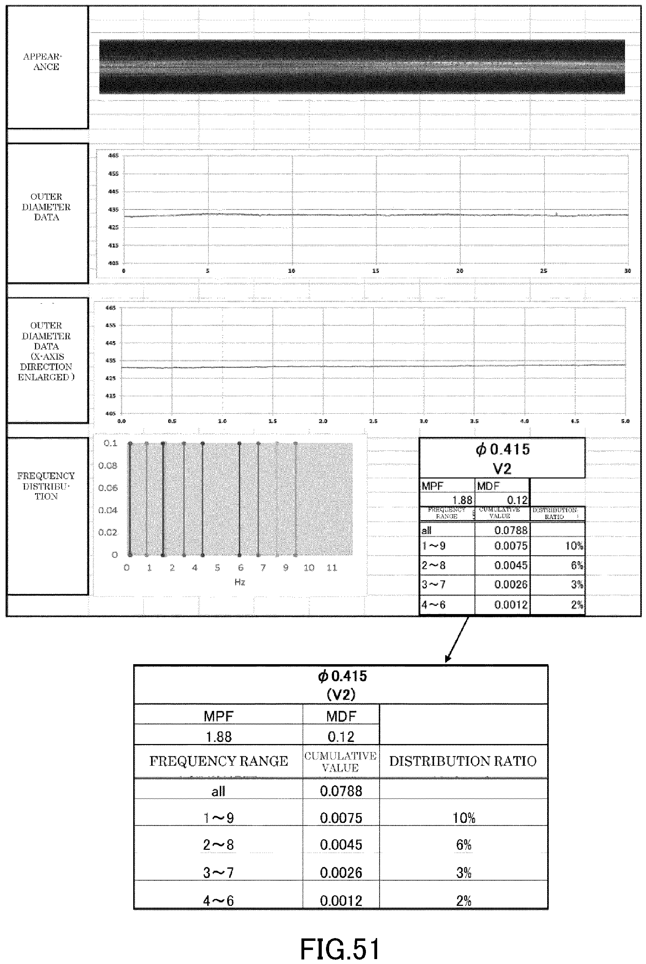

FIG. 51 is an enlarged external view and data for a case where a guide wire having a diameter of 0.415 mm is resin-coated at a coating speed V2. The MPF of the guide wire is 1.88, and the MDF is 0.12.

The resin coating speed will be described. The resin coating speed is the rate at which the resin film that forms the outer layer part 12 is formed on the surface of the guide wire (or the catheter). In the present example, the coating speed V1 is the fastest, and the coating speed V2 is the slowest. The coating speeds V3, V4 and V5 are located between the coating speeds V1 and V2, and the relationship is V1 > V3 > V4 > V5 > V2. The experimental results of Example 5 show that when the coating speed is V2 or less, the recesses 13 are not formed on the surface of the outer layer part 12. It can be seen that the recesses 13 are more easily formed as the coating speed exceeds V2 and becomes faster. Therefore, in Example 5, for example, it is disclosed that "irregularly disposed recesses 13 that are formed based on cracks generated by coating a resin suspension on the surface of the base material part 11 at a predetermined speed or higher, and then drying the resin suspension" are provided.

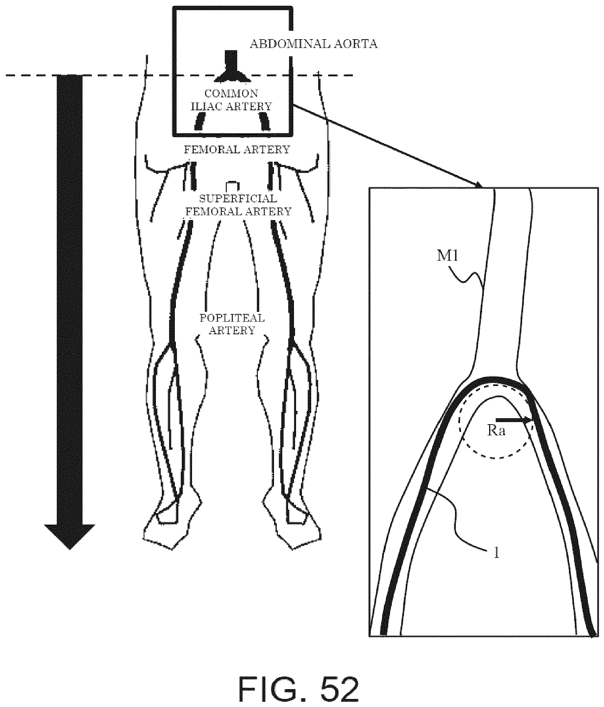

FIG. 52 is an explanatory diagram showing an example of a blood vessel in which a guide wire is used. The simulated blood vessel model M1 is formed to imitate, for example, a state in which a curved lesion has occurred in the common iliac artery. Here, the curvature Ra is referred to as a gently curved portion. A simulated blood vessel that realizes a gently curved portion can be referred to as a gently curved simulation Ra.

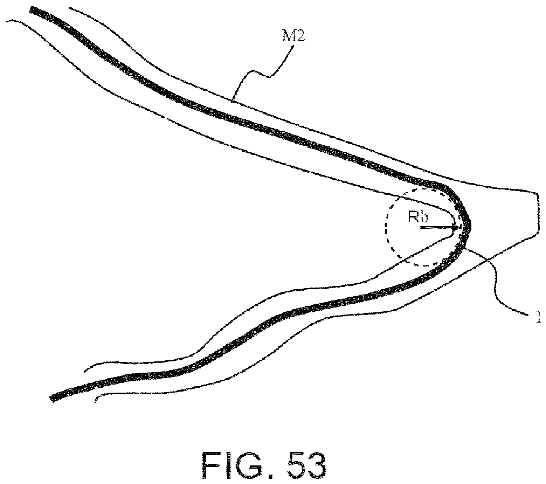

FIG. 53 shows a lower extremity blood vessel model M2 in which a strongly curved portion Rb is formed. The radius of curvature of the strongly curved portion Rb is smaller than the radius of curvature of the gently curved portion Ra (Rb < Ra).

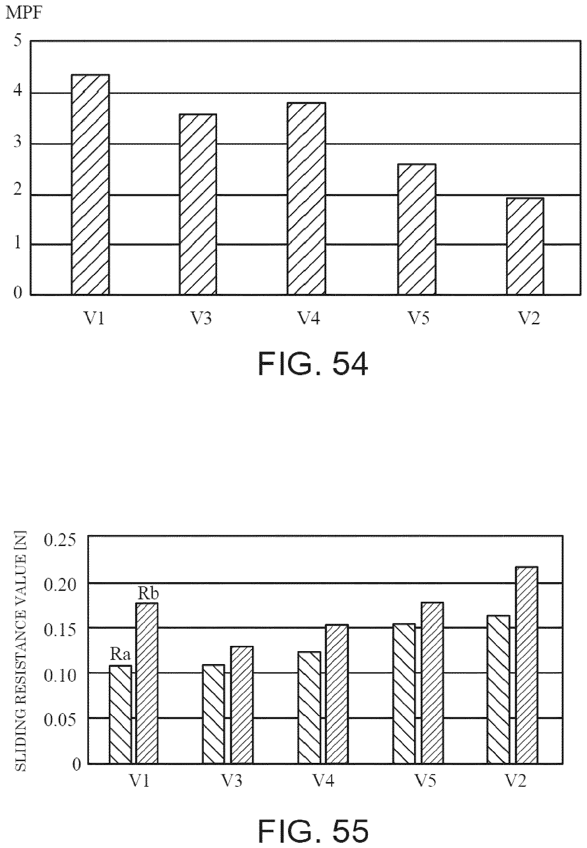

FIG. 54 is a graph showing the difference in MPF for cases where the resin coating speed with respect to a 0.415 mm guide wire is varied.

FIG. 55 is a graph showing, for each resin coating speed of a 0.415 mm guide wire, a comparison between the sliding resistance value when used in a location where the simulated blood vessel is strongly curved, and the sliding resistance value when used in a location where the simulated blood vessel is gently curved.

From FIG. 54 and FIG. 55, it can be seen that as the coating speed increases, the MPF value increases and the sliding resistance value decreases. Further, there is a negative correlation between the MPF and the sliding resistance value. The MPF represents the average frequency, and is for detecting the frequency as the number of wrinkles (recesses and protrusions). Therefore, a large MPF value indicates a large number of wrinkles, and the sliding properties are considered to improve as the number of wrinkles increases.

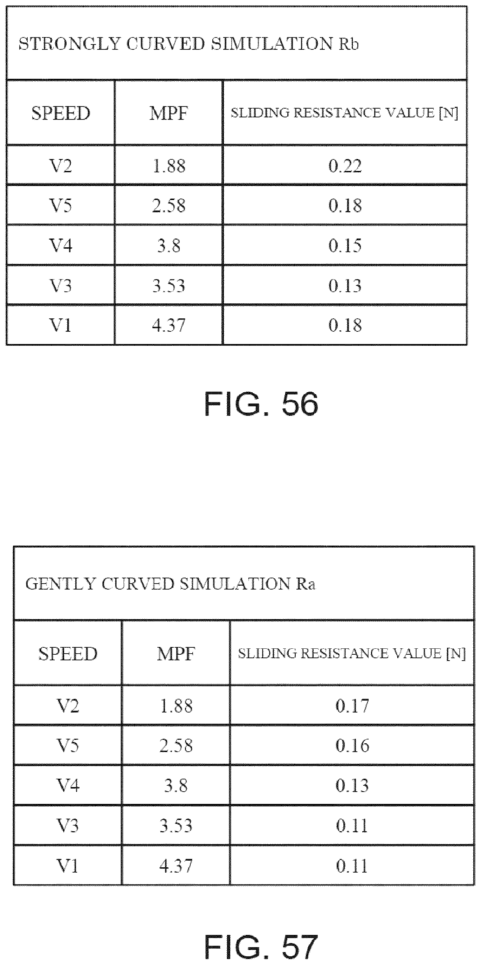

FIG. 56 is a table showing the MPF of a plurality of guide wires prepared with varying resin coating speeds, and the sliding resistance values of the plurality of guide wires when used in a strongly curved portion Rb of a simulated blood vessel.

FIG. 57 is a table showing the MPF of a plurality of guide wires prepared with varying resin coating speeds, and the sliding resistance values of the plurality of guide wires when used in a gently curved portion Ra of a simulated blood vessel.

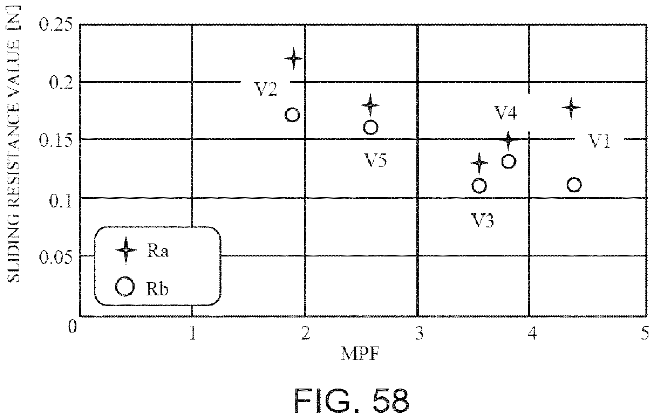

FIG. 58 is a graph comparing the measurement data in FIG. 56 and the measurement data in FIG. 57 according to the type of curvature Ra and Rb of the simulated blood vessel.

From FIG. 56 to FIG. 58, it can be seen that the sliding resistance value of the guide wire in the gently curved portion Ra becomes smaller than the sliding resistance value of the guide wire in the strongly curved portion Rb.

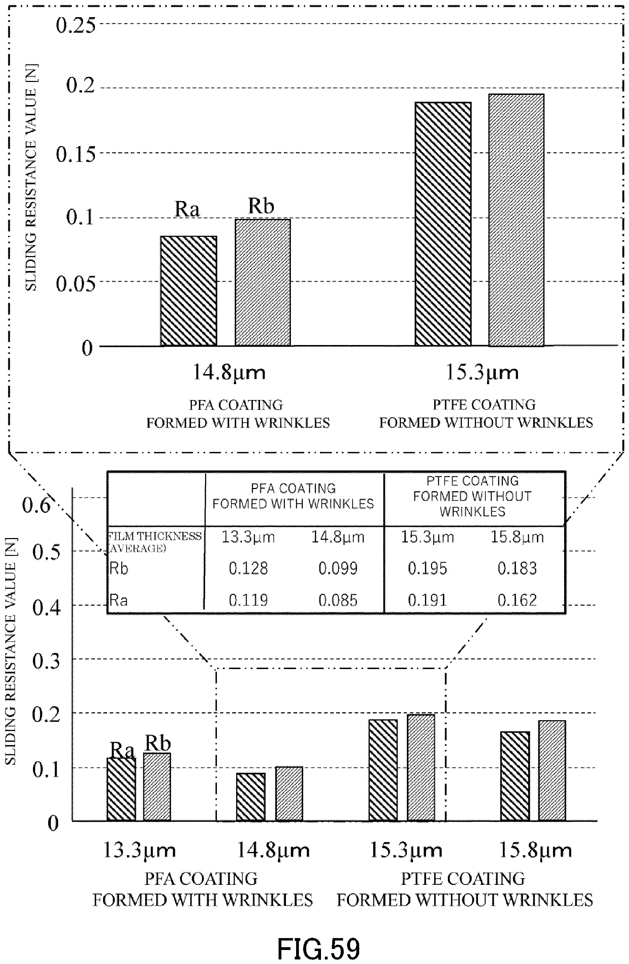

FIG. 59 is a graph showing a comparison between the sliding resistance value when a guide wire is coated with PFA and the sliding resistance value when coated with PTFE.

In FIG. 59, the sliding resistance values of a PFA-coated guide wire having wrinkles (recesses 13) on the surface and a PTFE-coated guide wire not having wrinkles on the surface are compared according to the strength of the curved portion.

The average value of the PFA coating thickness is shown for a 13.3 pm guide wire and a 14.8 pm guide wire. The average value of the PTFE coating thickness is shown for a 15.3 pm guide wire and a 15.8 pm guide wire.

From FIG. 59, it can be seen that, by forming wrinkles (recesses 13) on the surface of the resin layer that forms the outer layer part 12, the sliding resistance value of the PFA-coated guide wire formed with wrinkles becomes lower than the sliding resistance value of the PTFE-coated guide wire formed without wrinkles. Further, the coefficient of dynamic friction, which is a mechanical property of the fluororesin, is greater for a PFA-coated guide wire formed without wrinkles than for a PTFE-coated guide wire formed without wrinkles.

Note that, in FIG. 59, although a comparison is made between a PFA resin layer formed with wrinkles on the surface and a PTFE resin layer formed without wrinkles as a comparative example, the present disclosure does not exclude formation of wrinkles and reduction of the sliding resistance in resin layers other than PFA. These are also included in the scope of the present disclosure.

### [Example 6]

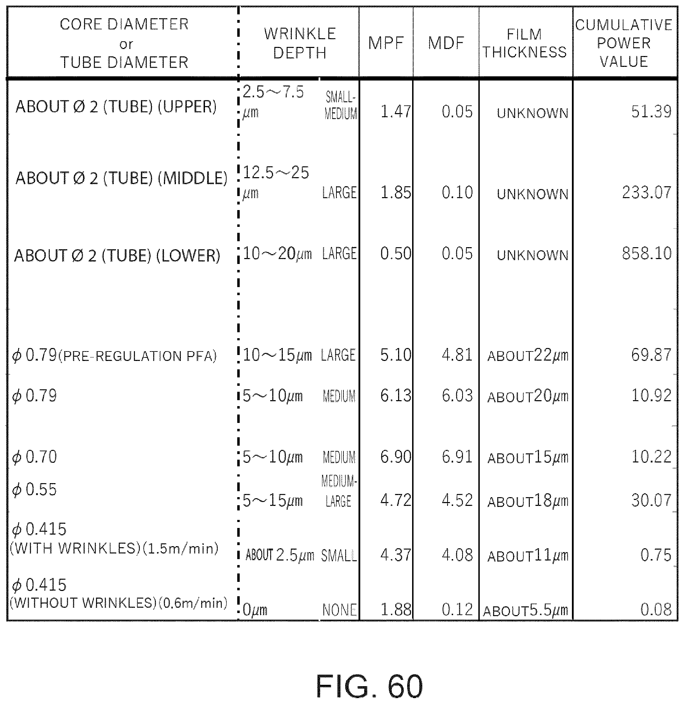

Example 6 will be described using FIG. 60 to FIG. 78. In Example 6, the results of a case where the recesses 13 are irregularly formed on the surface of a catheter will be described.

FIG. 60 is a table showing the depth dimension of the recesses (wrinkle depth), MPF, MDF, film thickness, and cumulative power value for the catheter according to Example 6.

In this table, the wrinkle depth, MPF, MDF, film thickness, and cumulative power value for the near side of a medical tube (catheter) having a diameter of 2 mm, the middle portion of a medical tube (catheter) having a diameter of 2 mm, the far side of a medical tube (catheter) having a diameter of 2 mm, a guide wire having a diameter of 0.79 mm, a guide wire having a diameter of 0.70 mm, a guide wire having a diameter of 0.55 mm, a guide wire having a diameter of 0.415 mm (with wrinkles on the surface), and a guide wire having a diameter of 0.415 mm (without wrinkles on the surface). The data for the guide wires is the same as that shown in FIG. 42.

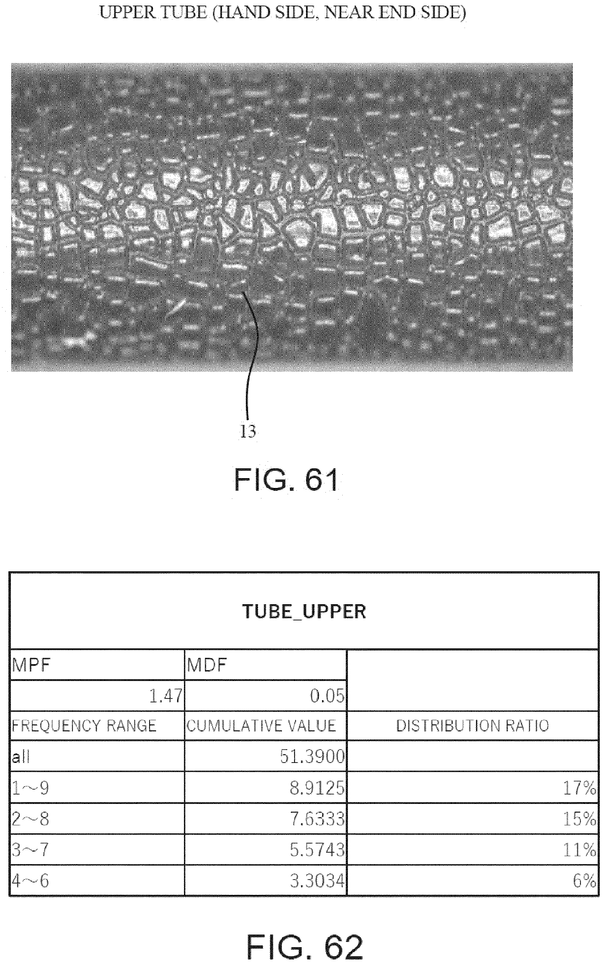

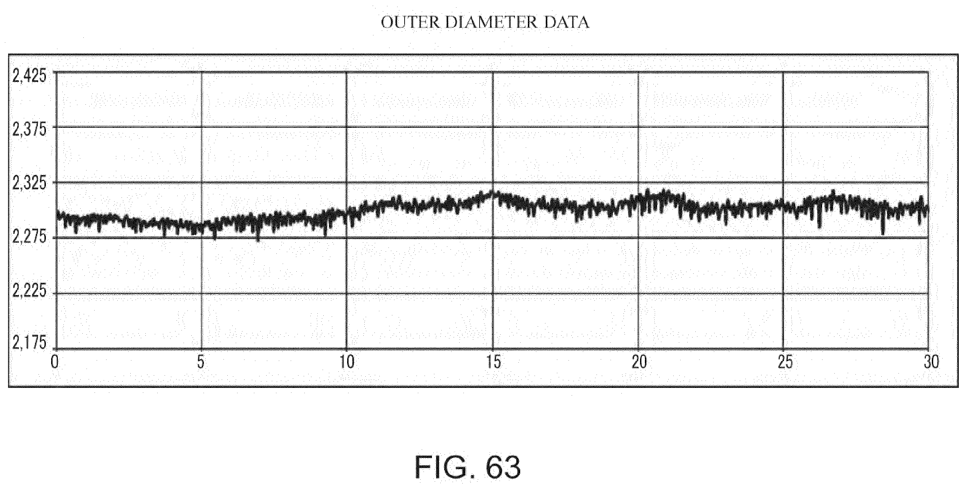

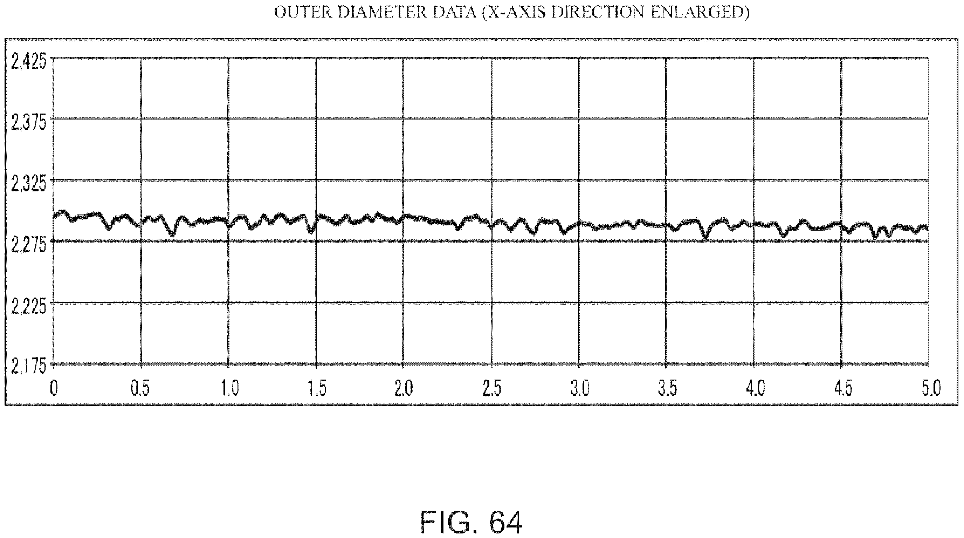

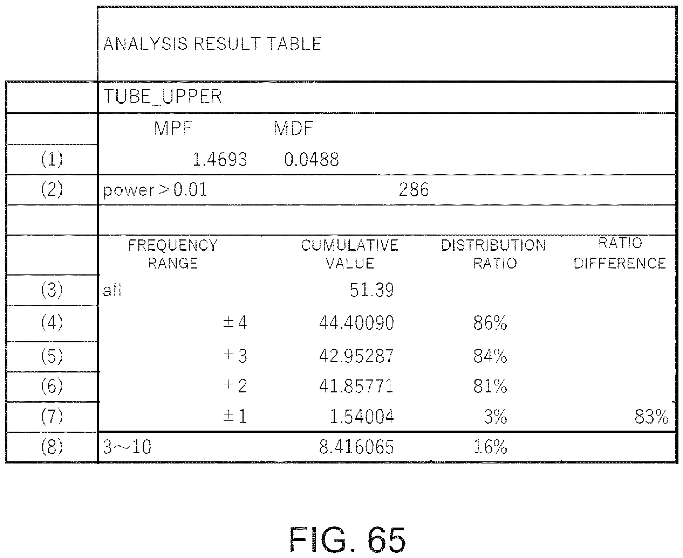

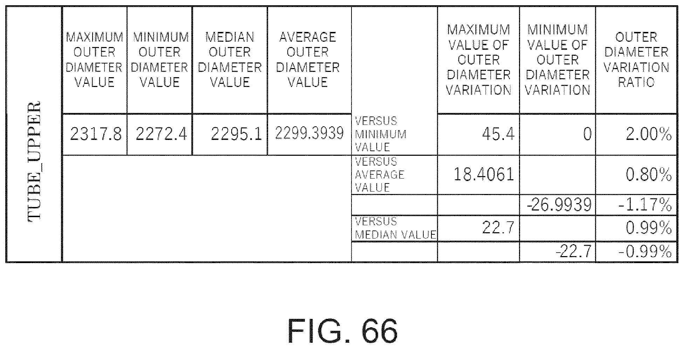

FIG. 61 is an enlarged view of the exterior of the near side of a catheter (labeled as tube_upper). It can be seen that the recesses 13 are formed in a mesh pattern. FIG. 62 is a table showing MDF and MPF values. FIG. 63 is a graph showing the outer diameter of a catheter along the length direction of the catheter. FIG. 64 is a graph showing a portion of FIG. 63 enlarged. FIG. 65 is a table showing analysis results of data for the near side of a catheter. FIG. 66 is a table showing measurement results of outer diameter data for the near side of a catheter.

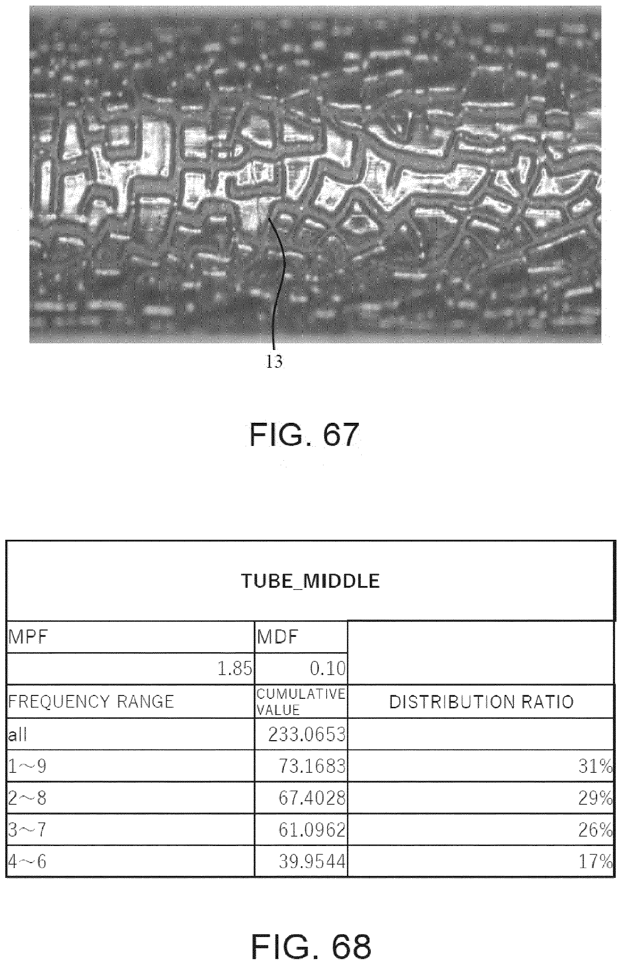

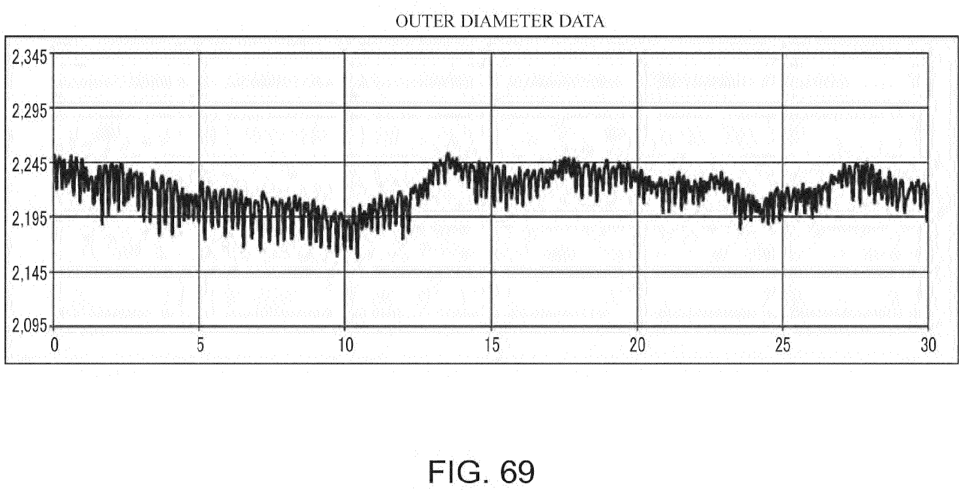

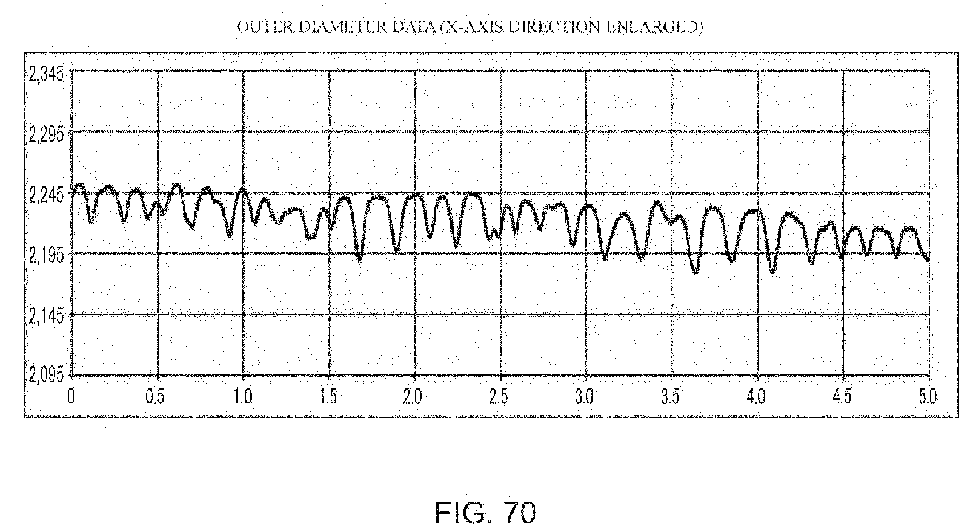

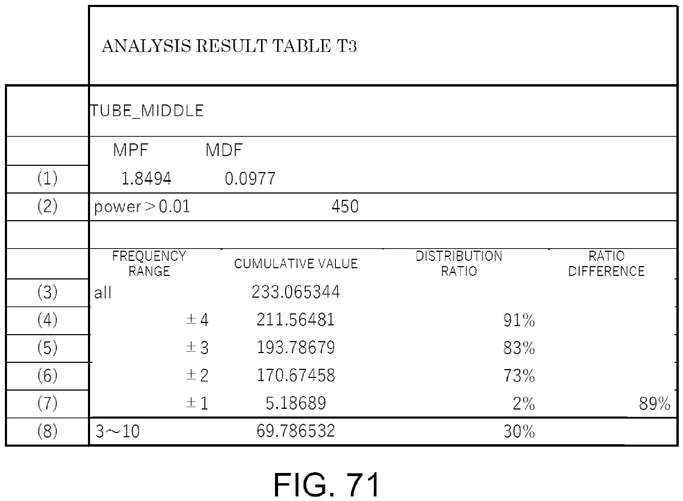

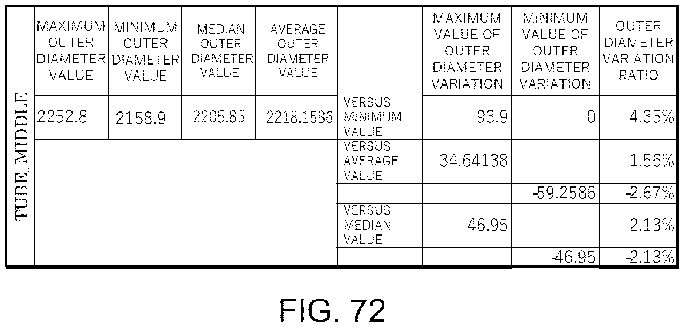

FIG. 67 is an enlarged view of the exterior near the middle of a catheter (labeled as tube_middle). FIG. 68 is a table showing MDF and MPF values. FIG. 69 is a graph showing the outer diameter of a catheter along the length direction of the catheter. FIG. 70 is a graph showing a portion of FIG. 69 enlarged. FIG. 71 is a table showing analysis results of data near the middle of a catheter. FIG. 72 is a table showing measurement results of outer diameter data near the middle of a catheter.

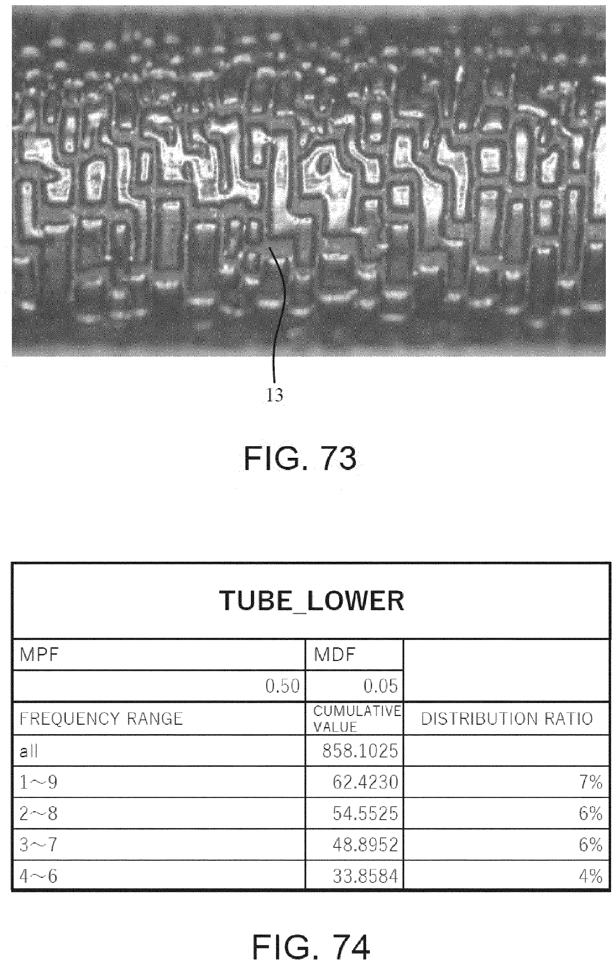

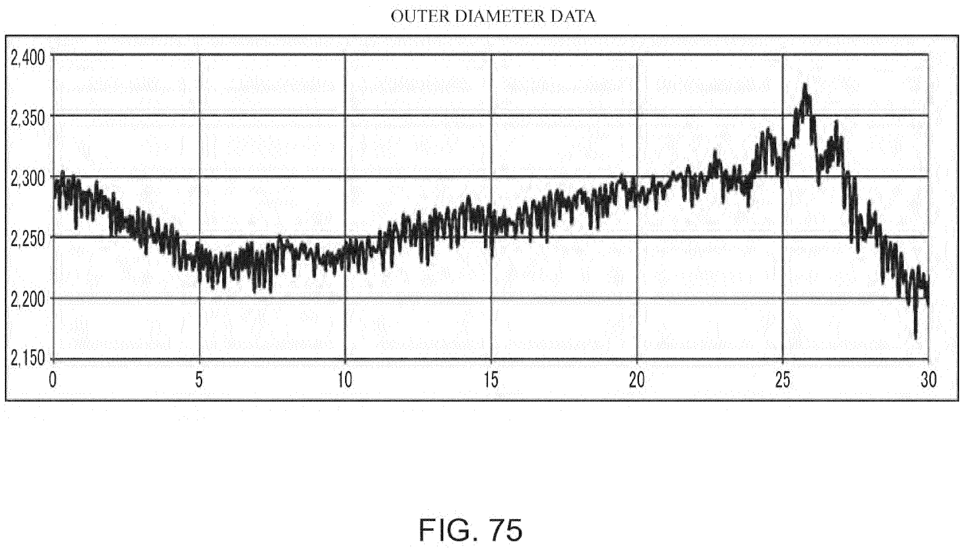

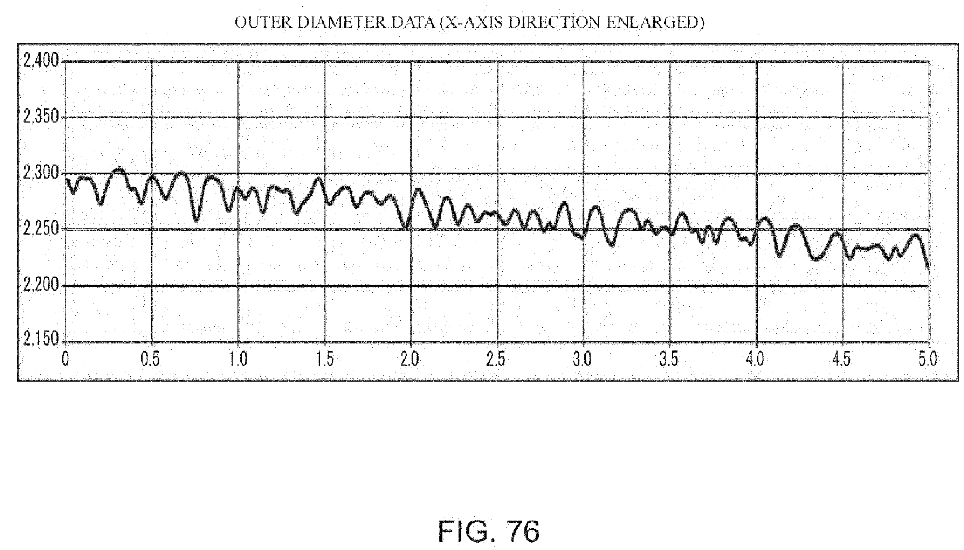

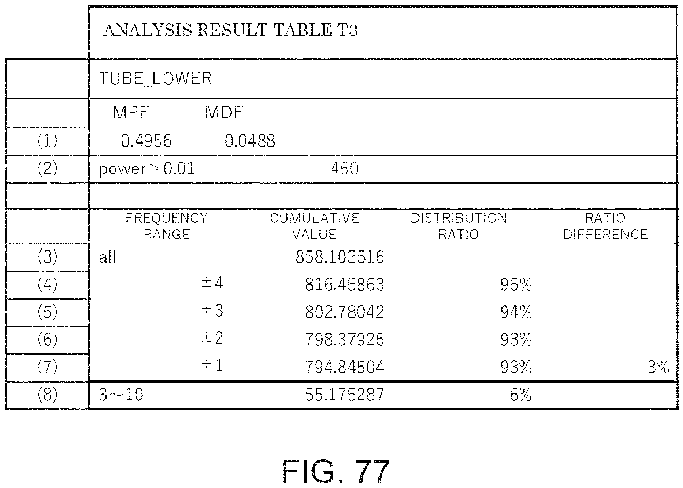

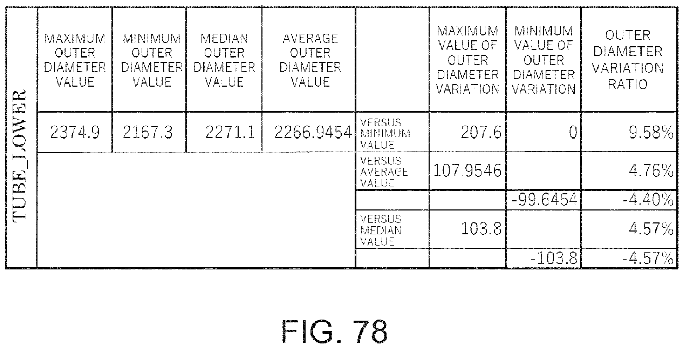

FIG. 73 is an enlarged view of the exterior of the far side of a catheter (labeled as tube_lower). FIG. 74 is a table showing MDF and MPF values. FIG. 75 is a graph showing the outer diameter of a catheter along the length direction of the catheter. FIG. 76 is a graph showing a portion of FIG. 75 enlarged. FIG. 77 is a table showing analysis results of data for the far side of a catheter. FIG. 78 is a table showing measurement results of outer diameter data for the far side of a catheter.

Although the disclosed embodiments have been described above, the present disclosure is not limited to the aforementioned embodiments and can be variously modified.

The embodiments described above have been described in detail to enable the disclosed embodiments to be understood more easily, and are not necessarily limited to those having all the described configurations. Furthermore, the configuration of an embodiment may be replaced with the configuration of another embodiment. Moreover, the configuration of another embodiment can be added to the configuration of an embodiment. In addition, portions of the configuration of each embodiment can be added, deleted, or replaced with other configurations.

Furthermore, the technical features included in the embodiments described above are not limited to the combinations explicitly stated in the claims, and can be combined as appropriate.

The present disclosure includes the configurations expressed as follows.

### (Expression 1)

A elongated medical instrument comprising: a base material part; an outer layer part made of a resin provided on a surface of the base material part; wherein a substantially flat surface of the outer layer part has irregularly disposed recesses or protrusions.

### (Expression 2)

The elongated medical instrument according to expression 1, wherein the substantially flat surface is a surface corresponding to a region of a lower side of the outer layer part on which a coil is not disposed.

### (Expression 3)

The elongated medical instrument according to expression 1, wherein a shape of the recesses is an irregularly-shaped linear portion.

### (Expression 4)

The elongated medical instrument according to expression 3, wherein a waveform shape exhibited by an outer diameter having the recesses or the protrusions includes a spatial frequency of 3 to 10 (l/mm).

### (Expression 5)

A elongated medical instrument comprising: a base material part; an outer layer part made of a resin provided on a surface of the base material part; wherein a substantially flat surface of the outer layer part has irregularly disposed recesses or protrusions, and at least one of conditions (1) to (9) below are satisfied when a measurement result of an outer diameter dimension along a longitudinal axis direction of the outer layer part is analyzed by FFT (Fast Fourier Transform).
(Condition 1) A value of at least either a median power frequency (referred to as MDF below) or a mean power frequency (referred to as MPF below) is 4 to 7 (l/mm)
(Condition 2) At least one of the MDF or the MPF is 5 (l/mm) or more
(Condition 3) There are 100 or more spatial frequencies in which the power value is 0.01 or more
(Condition 4) The spatial frequencies with a power value of 0.01 or more are distributed from 1 to 10 (l/mm)
(Condition 5) A distribution ratio in a range of an average frequency component value plus or minus 1 (l/mm) is less than 35%
(Condition 6) A distribution ratio in a range of an average frequency component value plus or minus 4 (l/mm) is approximately 70% or more
(Condition 7) A difference between a distribution ratio in a range of an average frequency component value plus or minus 1 (l/mm) and a distribution ratio of plus or minus 4 (l/mm) is approximately 40% or more
(Condition 8) A power spectrum of the spatial frequencies 3 to 10 (l/mm) is 50% or more
(Condition 9) A power spectrum of the spatial frequencies 3 to 10 (l/mm) is 65% or more

### (Expression 6)

A elongated medical instrument comprising: a base material part; an outer layer part made of a resin provided on a surface of the base material part; wherein a substantially flat surface of the outer layer part has irregularly disposed recesses or protrusions, and a measurement result of an outer diameter dimension along a longitudinal axis direction of the outer layer part satisfies at least one of conditions (10) or (11) below.
(Condition 10) An outer diameter variation ratio with respect to a minimum outer diameter value is 3% or more
(Condition 11) In a range of 1 cm in a longitudinal axis direction, there are ten or more locations in which a change in an outer diameter is 5 pm or more in a width of less than l/mm

### (Expression 7)

The elongated medical instrument according to expression 6, wherein in condition 10, in a case where the outer layer part includes a tapered portion, an outer diameter variation ratio excluding a change in an outer diameter due to the tapered portion is 3% or more with respect to a minimum outer diameter value.

### (Expression 8)

A elongated medical instrument according to any one of expressions 1 to 7, wherein the outer layer part is any one of PFA (perfluoroalkoxyalkane), FEP (tetrafluoroethylene-hexafluoropropylene copolymer), or ETFE (tetrafluoroethylene-ethylene copolymer).

### (Expression 9)

A elongated medical instrument according to any one of expressions 1 to 7, wherein the elongated medical instrument is a guide wire.

### (Expression 10)

The elongated medical instrument according to expression 9, wherein an outer diameter dimension of the guide wire is 1/mm or less.

### (Expression 11)

The elongated medical instrument according to expression 9, wherein an outer diameter dimension of the guide wire is 700 pm or less.

### (Expression 12)

The elongated medical instrument according to expression 9, wherein an average value of an outer diameter dimension of the guide wire is 500 to 600 pm.

### (Expression 13)

A elongated medical instrument according to any one of expressions 1 to 7, wherein the elongated medical instrument is a catheter.

### DESCRIPTION OF REFERENCE NUMERALS

1, 1A, 1B Guide wire,
1CE Guide wire according to comparative example,
2 Catheter,
11 Base material part,
12 Outer layer part,
13 Recess,
14 Protrusion,
111A, 111B Tubular body,
112A, 112B Intermediate resin layer,
113A, 113B Adhesive layer,
130 Crack

## Claims

1. An elongated medical instrument comprising:
a base material part; and
an outer layer part made of a resin based on a resin suspension that is coated on an outer peripheral surface of the base material part, a surface of the outer layer part comprising:
irregularly disposed recesses formed from cracks generated by drying the resin suspension; and
protrusions with an outer peripheral surface that is formed at an outer periphery of the recesses.

2. An elongated medical instrument comprising:
a base material part; and
an outer layer part made of a resin and provided on an outer peripheral surface of the base material part, a surface of the outer layer part comprising:
irregularly disposed recesses; and
protrusions with an outer peripheral surface that is formed at an outer periphery of the recesses;
wherein:
the outer layer part is formed by drying a resin suspension after being coated on a surface of the base material part, and
the recesses are formed by drying a suspension coated at the outer layer part.

3. The elongated medical instrument according to claim 1 or 2, wherein the recesses are disposed linearly and irregularly in a circumferential direction and in a longitudinal direction.

4. The elongated medical instrument according to any one of claims 1 to 3, wherein the resin suspension comprises fluororesin particles.

5. The elongated medical instrument according to claim 4, wherein the fluororesin particles are perfluoroalkoxyalkane particles.

6. The elongated medical instrument according to any one of claims 1 to 5, wherein the elongated medical instrument is a guide wire comprising:
the base material part,
a first region in which a coil member disposed on the outer peripheral surface of the base material part, and
a second region in which the outer layer part is disposed on the outer peripheral surface of the base material part, the second region being proximal to the first region, and
the recesses are provided in the second region.

7. The elongated medical instrument according to claim 1, wherein:
the base material part comprises:
a tubular body; and
a reinforcing body formed on an outer peripheral side of the tubular body, and
the outer layer part covers the tubular body and the reinforcing body.

8. The elongated medical instrument according to claim 7, comprising:
an adhesive layer provided on and between an outer peripheral side of the reinforcing body and the outer layer part, and made of a resin increasing an adhesion with the reinforcing body.

9. The elongated medical instrument according to claim 8, wherein the resin increasing the adhesion is a fluororesin having an adhesive functional group.

10. The elongated medical instrument according to claim 9, wherein the fluororesin having an adhesive functional group is a perfluoroalkoxyalkane having an adhesive functional group.

11. The elongated medical instrument according to claim 1, wherein:
the base material part comprises:
a tubular body;
a reinforcing body formed on an outer peripheral side of the tubular body; and
an intermediate resin layer formed so as to cover the tubular body and bury the reinforcing body, and
the outer layer part covers the tubular body and the reinforcing body.

12. The elongated medical instrument according to claim 11, wherein:
the intermediate resin layer and the outer layer part are formed of different resins, and
an adhesive layer made of a resin having increased adhesion with the intermediate resin layer is formed between the intermediate resin layer and the outer layer part.

13. The elongated medical instrument according to claim 12, wherein the resin forming the adhesive layer is a fluororesin having an adhesive functional group.

14. The elongated medical instrument according to claim 9, wherein the fluororesin having an adhesive functional group is a perfluoroalkoxyalkane having an adhesive functional group.

15. A method for producing an elongated medical instrument, the method comprising:
a first step of providing a base material part;
a second step of coating a resin suspension on an outer periphery of the base material part;
a third step of drying the resin suspension coated on the base material part; and
a fourth step of sintering a coating of the resin suspension;
wherein:
in the third step, cracks are formed in the coating of the dried resin suspension, and
after the fourth step, recesses are formed in a surface due to the cracks.

16. The method according to any one of claims 15 to 17, further comprising:
between the first step and the second step, a fifth step of forming an adhesive layer with a resin increasing adhesion to the base material part.
